# EUROPEAN PATENT SPECIFICATION

(11) **EP 1 546 195 B1**
(45) Date of publication and mention of the grant of the patent: **31.10.2007**
(21) Application number: 03792443.8
(22) Date of filing: 22.08.2003
(51) Int. Cl.: C07K 14/465

(54) **IMPROVED MUTANTS OF BIOTIN BINDING PROTEIN**
VERBESSERTE MUTANTEN VON BIOTINBINDENDEM PROTEIN
MUTANTS AMELIORES DE PROTEINES FIXANT LA BIOTINE

(30) Priority: 23.08.2002 FI 20021518
(43) Date of publication of application: 29.06.2005
(73) Proprietor: Jyväskylän Yliopisto, 40351 Jyväskylä (FI)
(72) Inventor: KULOMAA, Markku, Sakari, FIN-33200 Tampere (FI); NORDLUND, Henri, Rainer, FIN-37560 Lempäälä (FI); LAITINEN, Olli-Heikki, FIN-33270 Tampere (FI); HYTÖNEN, Vesa, FIN-33920 Pirkkala (FI)
(74) Representative: Risku, Ira Marjatta
(86) International application number: PCT/FI2003/000619
(87) International publication number: WO 2004/018509

(56) References cited:
- WO-A1-01/05977
- WO-A1-97/11183
- NORDLUND HENRI R. ET AL.: 'Enhancing the thermal stability of avidin' THE JOURNAL OF BIOLOGICAL CHEMISTRY vol. 278, no. 4, 2003, pages 2479 - 2483, XP002973647
- REZNIK GABRIEL O. ET AL.: 'Streptavidins with intersubunit crosslinks have enhanced stability' NATURE BIOTECHNOLOGY vol. 14, 1996, pages 1007 - 1011, XP002926127
- MARTTILA ARI T. ET AL.: 'Recombinant neutralite avidin: a non-glycosylated, acidic mutant of chicken avidin that exhibits high affinity for biotin and low non-specific binding properties' FEBS LETTERS vol. 467, 2000, pages 31 - 36, XP004260917
- LAITINEN OLLI H. ET AL.: 'Chicken avidin-related proteins show altered biotin-binding and physico-chemical properties as compared with avidin' BIOCHEM. J. vol. 363, 2002, pages 609 - 617, XP002973648
- MASAZUMI MATSUMURA ET AL.: 'Substantial increase of protein stability by multiple disulphide bonds' NATURE vol. 342, 16 November 1989, pages 291 - 293, XP002973649
- PACE C. NICK ET AL.: 'Conformational stability and activity of ribonuclease T1 with Zero, One and Two intact disulfide bonds' THE JOURNAL OF BIOLOGICAL CHEMISTRY vol. 263, no. 24, 1988, pages 11820 - 11825, XP002973650
- CREIGHTON THOMAS E.: 'Disulphide bonds and protein stability' BIOESSAYS vol. 8, no. 2, 1988, pages 57 - 63, XP002973651
- BAYER EDWARD A. ET AL.: 'Preparation of deglycosylated egg white avidin' APPLIED BIOCHEMISTRY AND BIOTECHNOLOGY vol. 53, 1995, pages 1 - 9, XP002973652

### SEQUENCE LISTING

<110> Jyväskylän yliopisto
<120> Improved mutants of biotin binding protein
<130> BP104962
<150> FI20021518
   <151> 2002-08-23
<160> 9
<170> PatentIn version 3.1
<210> 1
   <211> 420
   <212> DNA
   <213> Gallus gallus
   <221> Avidin
<400> 1
   gaattccgca aggagcacac ccggctgtcc acctgctgca gagatggtgc acgcaacctc      60
   cccgctgctg ctgctgctgc tgctcagcct ggctctggtg gctcccggcc tctctgccag     120
   aaagtgctcg ctgactggga aatggaccaa cgatctgggc tccaacatga ccatcggggc     180
   tgtgaacagc agaggtgaat tcacaggcac ctacatcaca gccgtaacag ccacatcaaa     240
   tgagatcaaa gagtcaccac tgcatgggac acaaaacacc atcaacaaga ggacccagcc     300
   cacctttggc ttcaccgtca attggaagtt ttcagagtcc accactgtct tcacgggcca     360
   gtgcttcata gacaggaatg ggaaggaggt cctgaagacc atgtggctgc tgcggtcaag     420
<210> 2
   <211> 540
   <212> DNA
   <213> Streptomyces avidinii
   <221> Streptavidin
<400> 2
   ccctccgtcc ccgccgggca acaactaggg agtatttttc gtgtctcaca tgcgcaagat      60
   cgtcgttgca gccatcgccg tttccctgac cacggtctcg attacggcca gcgcttcggc     120
   agacccctcc aaggactcga aggcccaggt ctcggccgcc gaggccggca tcaccggcac     180
   ctggtacaac cagctcggct cgaccttcat cgtgaccgcg ggcgccgacg gcgccctgac     240
   cggaacctac gagtcggccg tcggcaacgc cgagagccgc tacgtcctga ccggtcgtta     300
   cgacagcgcc ccggccaccg acggcagcgg caccgccctc ggttggacgg tggcctggaa     360
   gaataactac cgcaacgccc actccgcgac cacgtggagc ggccagtacg tcggcggcgc     420
   cgaggcgagg atcaacaccc agtggctgct gacctccggc accaccgagg ccaacgcctg     480
   gaagtccacg ctggtcggcc acgacacctt caccaaggtg aagccgtccg ccgcctccat     540
<210> 3
   <211> 1335
   <212> DNA
   <213> Gallus gallus
   <221> AVR1
<400> 3
   gaattctagg gacccgatcc acctgctggt gagtttgata ttcgtctctg gtcttcaatt      60
   ttggggttgt gcgttcaact ggaaaacgtg acccacccag attgcgtaac acctgggaag     120
   aaaagcctgc gccgggagca ataaaaggcg agggagcagg caggaggggt gagtcctgca     180
   aggagcacac ccggctgtcc acctgctgca gagatggtgc acgcaacctc cccgctgctg     240
   ctgctgctgc tgctcagcct ggctctggtg gctcctggcc tctctgccag aaaggtaatg     300
   gggtggggct gggagtgggt gcacctggtg cccacccctg cctcctgccc gccactgact     360
   ccttcttctt cgtcacagtg ctcgctgact gggaaatggg acaacgacct gggctccatc     420
   atgaccatcg gagctgtgaa cgacaatggc gagttcaatg gcacctacat cacagctgta     480
   gcagataatc caggaaacat cacgcgatca ccactgcttg ggatccaaca caaaagagcc     540
   tgccagccca cctttggctt cactgtccat tggaactttt caggtgcttc tctcccagcc     600
   tccctgcagt gtccctgctc ctctgctgtg cttccctgtg acaaacccct ttgctttcct     660
   gcccttcccc acgctgtctc cagtgctcgc ctgcccttcc ctacagactc cctgacggtc     720
   tctcctcctc actgtggtgt ccctgatgat ttccagccca tccctgcagt cccctcaaca     780
   atgccctgcc tcccatgccc ccggtgctgc cccatccctt cccgtagagc tgctgggctg     840
   ctgtcacctc ctggtccccg ggtgcagggg aggtgctggg gctgtcccca gagggcacgg     900
   agagctcaga tgagttgtcc cctgggcaga gggaccgtgg tgctggcact gccctgccct     960
   gcgtggggct cacaacccca ctcccctcat ctgccccttt tcccaacaga gtccaccagt    1020
   gtctttgtgg gccagtgctt cgtggacaag agtggaaagg aggtcctgaa gaccaaatgg    1080
   ctgcaacggt tagcagttga tgacattagt gatgactgga aagctaccag gtgagcccag    1140
   ggcagaggca cacggtccca ggctgtgact cgatggctgt gcacttccca ccttacatct    1200
   cctctctctc cccgcagggt cggcaacaac gacttcactc gccagcgcac agtggaggag    1260
   tgaggatggc cccgcaaagc cagcaacaat gccggagtgc tgacactgct tgtgatattc    1320
   ctcccaataa agctt                                                     1335
<210> 4
   <211> 1335
   <212> DNA
   <213> Gallus gallus
   <221> AVR2
<400> 4
   gaattctaga gacccgatcc acctgctggt gagtttgata ttcgtctctg gtcttcaatt      60
   ttggggttgt gcgttcaact ggaaaacgtg acccacccag attgcgtaac acctgggaag     120
   aaaagcctgc gccgggagca ataaaaggcg agggagcagg caggaggggt gagtcctgca     180
   aggagcacac ccggctgtcc acctgctgca gagatggtgc acgcaacctc cccgctgctg     240
   ctgctgctgc tgctcagcct ggctctggtg gctcccagcc tctctgccag aaaggtaacg     300
   ggatggggct gggagtgggt gcacctggtg cccaccactg cctcctgccc gccactgact     360
   ccttcttctt cactgcagtg ctcgctgact ggggaatggg acaacgacct gggctccatc     420
   atgaccatcg gagctgtgaa cgacaatggc gagttcgatg gcacctacat cacagctgta     480
   gcagataatc caggaaacat cacgctatca ccactgcttg ggatccaaca caaaagagcc     540
   agccagccca cttttggctt cactgtccat tggaactttt caggtgcttc tctcccagcc     600
   tccctgcaat gtccctgctc ctctgctgtg cttccctgtg acaaacccct ctgctttcct     660
   gcccttcccc acgctgtctc cagtgctctc ctgcccttcc ctacagtctc cctgacggtc     720
   tctcctcctc gctgtggtgt ccctgatgat ttccagccct tccctgcaat cccctcaaca     780
   atgccctgcc tcccatgccc ccggtgctgc cccatccctt cccgtagagc tgctgggctg     840
   ctgtcacctc caggtccccg ggtgcagggg aagtgctggg gctgtcccca gagggcacag     900
   agagctcaga tgagttgtcc cctgggcaga gggaccatgg cactggcact gccctgccct     960
   gcgtggggct cacaacccca ctcccctcat ctgccccttt tcccaacaga gtccaccagt    1020
   gtctttgtgg gccagtgctt cgtggacagg agcggaaagg aggtcctgaa gaccaaatgg    1080
   ctgcaacggt tagcagttga tgacattagt gatgactgga tagctaccag gtgagcccag    1140
   ggcagaggca cacggtcccg ggctgtgact cgatggctgt gcacttccca ccttacatct    1200
   cctctctctc cccgcagggt cggcaacaac gacttcactc gccagcacac agtggaagag    1260
   tgaggatggc cccgcaaagc cagcaacaat gccagagtgc tgacactgct tgtgatattc    1320
   ctcccaataa agctt                                                    1335
<210> 5
   <211> 1133
   <212> DNA
   <213> Gallus gallus
   <221> AVR3
<400> 5
   cctgctgcag agatggtgca cacaacctcc ccgctgctgc tgctgctgct gctcagcctg      60
   gctctggtgg ctcccagcct ctctgccaga aaggtaacgg gatggggctg ggagtgggtg     120
   cacctggtgc ccaccactgc ctcctgcccg ccactgactc cttcttcttc attgcagtgc     180
   tcgctgactg ggaaatggac caacaacctg ggctccatca tgaccatcag ggctgtgaac     240
   agcagaggtg aattcgcagg cacctacctc acagctgtag cagataatcc aggaaacatc     300
   aagctatcac cactgcttgg gatccaacac aaaagagcct gccagcccac ctttggcttc     360
   actgtccatt ggaacttttc aggtgcttct ctcccagcct ccctgcagtg tccctgctcc     420
   tctgctgtgc ttccctgtga caaacccctc tgctttcctg cccttcccca cgctgtctcc     480
   agtgctctcc tgcccttccc tacagtctcc ctgacggtct ctcctcctcg ctgtggtgtc     540
   cctgatgatt tccagctcat ccctgcaatc ccctcaacaa tgccctgcct cccatgcccc     600
   cggtgctgcc ccatcccttc ccgtagagct gctgggctgc tgtcacctcc tggtccccgg     660
   gtgcagggga ggtgctgggg ctgtccccag agggcacaga gagctcagat gagttgtccc     720
   ctgggcagag ggaccgtggt gctggcactg tcctgccctg cgtggggctc acgaccccac     780
   tcccctcatc tgcccctttt cccacagagt ccaccagtgt ctttgtgggc cagtgcttca     840
   tagacaggag cggaaaggag gtcctgaaga ccaaatggct gcaacggtta gcagttgatg     900
   acattagtga tgactggaaa gctaccaggt gagcccaggg cagaggcaca cggtcccggg     960
   ctgtgactcg atggctgtgc acttcccacc ttacatctcc tctctctccc cgcagggtcg    1020
   gctacaacaa cttcactcgc cagcgcacag tggaggagtg aggatggccc cgcaaagcca    1080
   gcaacaatgc cggagtgctg acactgcttg tgatattcct cccaataaag ctt           1133
<210> 6
   <211> 1334
   <212> DNA
   <213> Gallus gallus
   <221> AVR4
<400> 6
   gaattctagg gacccgatcc acctgctggt gagtttgata ttcgtctctg gtcttcaatt      60
   ttggggttgt gcgttcaact ggaaaacgtg acccacccag attgcgtaac acctgggaag     120
   aaaagcctgc gccgggagca ataaaaggcg agggagcagg caggaggggt gagtcctgca     180
   aggagcacac ccggctgtcc acctgctgca gagatggtgc acacaacctc cccgctgctg     240
   ctgctgctgc tgctcagcct ggctctggtg gctcccagcc tctctgccag aaaggtaacg     300
   ggatggggct gggagtgggt gcacctggtg cccaccactg cctcctgccc gccactgact     360
   ccttcttctt cattgcagtg ctcgctgact gggaaatgga ccaacaacct gggctccatc     420
   atgaccatca gggctgtgaa cagcagaggt gaattcacag gcacctacct cacagctgta     480
   gcggataatc caggaaacat cacgctatca ccactgcttg ggatccaaca caaaagagcc     540
   agccagccca cctttggctt cactgtccat tggaactttt caggtgcttc tctcccagcc     600
   tccctgcagt gtccctgctc ctctgctgtg cttccctgtg acaaacccct ctgctttcct     660
   gcccttcccc acgctgtctc cagtgctctc ctgcccttcc ctacagtctc cctgacggtc     720
   tctcctcctc gctgtggtgt ccctgatgat ttccagccca tccctgcagt cccctcaaca     780
   atgccctgcc tcccatgccc ccggtgctgc cccatccctt cccgtagagc tgctgggctg     840
   ctgtcacctc caggtccccg ggtgcagggg aggtgctggg gctgtcccca gagggcacag     900
   agagctcaga tgagttgtcc cctgggcaga gggaccatgg cactggcact gccctgccct     960
   gcgtggggct cacaacccca ctcccctcat ctgccccttt tcccacagag tccaccactg    1020
   tcttcacggg ccagtgcttc atagacagga acgggaagga ggtcctgaag accatgtggc    1080
   tgctgcggtc aagtgttaat gacattagtt atgactggaa agctaccagg tgagcccagg    1140
   gcagaagcac acggtcccgg gctgtgactc aatggctgtg cacttcccac cttacatctc    1200
   ctcactctcc ccgcagggtc ggctacaaca acttcactcg cctgtgcaca gtggaggagt    1260
   gaggatggcc ccgcaaagcc agcaacaatg ccagagtgct gacactgctt gtgatattcc    1320
   tcccaataaa gctt                                                       1334
<210> 7
   <211> 1334
   <212> DNA
   <213> Gallus gallus
   <221> AVR5
<400> 7
   gaattctaga gacccgatcc acctgctggt gagtttgata ttcgtctctg gtcttcaatt 60
   ttggggttgt gcgttcaact ggaaaacgtg acccacccag attgcgtaac acctgggaag 120
   aaaagcctgc gccgggagca ataaaaggcg agggagcagg caggaggggt gagtcctgca 180
   aggagcacac ccggctgtcc acctgctgca gagatggtgc acacaacctc cccgctgctg 240
   ctgctgctgc tgctcagcct ggctctggtg gctcccagcc tctctgccag aaaggtaacg 300
   ggatggggct gggagtgggt gcacctggtg cccaccactg cctcctgccc gccactgact 360
   ccttcttctt cattgcagtg ctcgctgact gggaaatgga ccaacaacct gggctccatc 420
   atgaccatca gggctgtgaa cagcagaggt gaattcacag gcacctacct cacagctgta 480
   gcggataatc caggaaacat cacgctatca ccactgcttg ggatccaaca caaaagagcc 540
   agccagccca cctttggctt cactgtccat tggaactttt caggtgcttc tctcccagcc 600
   tccctgcagt gtccctgctc ctctgctgtg cttccctgtg acaaacccct ctgctttcct 660
   gcccttcccc acgctgtctc cagtgctctc ctgcccttcc ctacagtctc cctgacggtc 720
   tctcctcctc gctgtggtgt ccctgatgat ttccagccca tccctgcagt cccctcaaca 780
   atgccctgcc tcccatgccc ccggtgctgc cccatccctt cccgtagagc tgctgggctg 840
   ctgtcacctc caggtccccg ggtgcagggg aggtgctggg gctgtcccca gagggcacag 900
   agagctcaga tgagttgtcc cctgggcaga gggaccatgg cactggcact gccctgccct 960
   gcgtggggct cacaacccca ctcccctcat ctgccccttt tcccacagag tccaccactg 1020
   tcttcacggg ccagtgcttc atagacagga acgggaagga ggtcctgaag accatgtggc 1080
   tgctgcggtc aagtgttaat gacattagtt atgactggaa agctaccagg tgagcccagg 1140
   gcagaagcac acggtcccgg gctgtgactc aatggctgtg cacttcccac cttacatctc 1200
   ctcactctcc ccgcagggtc ggctacaaca acttcactcg cctgtgcaca gtggaggagt 1260
   gaggatggcc ccgcaaagcc agcaacaatg ccagagtgct gacactgctt gtgatattcc 1320
   tcccaataaa gctt 1334
<210> 8
   <211> 1133
   <212> DNA
   <213> Gallus gallus
   <221> AVR6
<400> 8
   cctgctgcag agatggtgca cgcaacctcc ccgctgctgc tgctgctgct gctcagcctg 60
   gctctggtgg ctcccggcct ctctgccaga aaggtaacgg gatggggctg ggagtgggtg 120
   cacctggcgc ccacccctgc ctcctgcctg ccactgactc cttcttcttc gtcacagtgc 180
   tcgctgactg gggaatggga caacaacctg ggctccatca tgaccatcgg agctgtgaac 240
   gacaatggcg agttcaatgg cacctacatc acagctgtag cagataatcc aggaaacatc 300
   aagctatcac cactgcttgg gatccaacac aaaagagcct gccagcccac ctttggcttc 360
   actgtccatt ggaacttttc aggtgcttct ctcccagcct ccctgcaatg tccctgctcc 420
   tctgctgtgc ttccctgtga caaacccctc tgctttcctg cccttcccca cgctgtctcc 480
   agtgctctcc tgcccttccc tacagtctcc ctgacggtct ctcctcctcg ctgtggtgtc 540
   cctgatgatt tccagcccat ccctgcagtc ccctcaacaa tgccctgcct cccatgcccc 600
   cggtgctgcc ccatcccttc ccgtagagct gctgggctgc tgtcacctcc tggtccccgg 660
   gtgcagggga ggtgctgggg ctgtccccag agggcacgga gagctcagat gagttgtccc 720
   ctgggcagag ggaccgtggt gctggcactg ccctgccctg cgtggggctc acaaccccac 780
   tcccctcatc tgcccctttt cccacagagt ccaccagtgt ctttgtgggc cagtgcttcg 840
   tggacaggag cggaaaggag gtcctgaaga ccaaatggct gcaacggtta gcagttgatg 900
   acattagtga tgactggaaa gctaccaggt gagcccaggg cagaggcaca cggtcccggg 960
   ctgtgactcg atggctgtgc acttcccacc ttacatctcc tctctctccc cgcagggtcg 1020
   gctacaacaa cttcactcgc cagcgcacag tggaggagtg aggatggccc cgcaaagcca 1080
   gcaacaatgc cggagtgctg acactgcttg tgatattcct cccaataaag ctt 1133
<210> 9
   <211> 1133
   <212> DNA
   <213> Gallus gallus
   <221> AVR7
<400> 9
   cctgctgcag agatggtgca cgcaacctcc ccgctgctgc tgctgctgct gctcagcctg 60
   gctctggtgg ctcccggcct ctctgccaga aaggtaacgg gatggggctg ggagtgggtg 120
   cacctggtgc ccacccctgc ctcctgcctg ccactgactc cttcttcttc gtcacagtgc 180
   tcgctgactg gggaatggga caacaacctg ggctccaaca tgaccatcgg agctgtgaac 240
   gacaatggcg agttcaatgg cacctacatc acagctgtag cagataatcc aggaaacatc 300
   aagctatcac cactgcttgg gatccaacac aaaagagcct gccagcccac ctttggcttc 360
   actgtccatt ggaacttttc aggtgcttct ctcccagcct ccctgcagtg tccctgctcc 420
   tctgctgtgc ttccctgtga caaacccctc tgctttcctg cccttcccca cgctgtctcc 480
   agtgctctcc tgcccttccc tacagtctcc ctgacggtct ctcctcctcg ctgtggtgtc 540
   cctgatgatt tccagctcat ccctgcaatc ccctcaacaa tgccctgcct cccatgcccc 600
   cggtgctgcc ccatcccttc ccgtagagct gctgggctgc tgtcacctcc tggtccccgg    660
   gtgcagggga ggtgctgggg ctgtccccag agggcacaga gagctcagat gagttgtccc    720
   ctgggcagag ggaccgtggt gctggcactg tcctgccctg cgtggggctc acgaccccac    780
   tcccctcatc tgcccctttt cccacagagt ccaccagtgt ctttgtgggc cagtgcttca    840
   tagacaggag cggaaaggag gtcctgaaga ccaaatggct gcaacggtta gcagttgatg    900
   acattagtga tgactggaaa gctaccaggt gagcccaggg cagaggcaca cggtcccggg    960
   ctgtgactcg atggctgtgc acttcccacc ttacatctcc tctctctccc cgcagggtcg   1020
   gctacaacaa cttcactcgc cagcgcacag tggaggagtg aggatggccc cgcaaagcca   1080
   gcaacaatgc cggagtgctg acactgcttg tgatattcct cccaataaag ctt          1133

### SEQUENCE LISTING

<110> Jyväskylän yliopisto
<120> Improved mutants of biotin binding protein
<130> BP104962
<150> FI20021518
   <151> 2002-08-23
<160> 9
<170> PatentIn version 3.1
<210> 1
   <211> 420
   <212> DNA
   <213> Gallus gallus
<400> 1
   gaattccgca aggagcacac ccggctgtcc acctgctgca gagatggtgc acgcaacctc     60
   cccgctgctg ctgctgctgc tgctcagcct ggctctggtg gctcccggcc tctctgccag    120
   aaagtgctcg ctgactggga aatggaccaa cgatctgggc tccaacatga ccatcggggc    180
   tgtgaacagc agaggtgaat tcacaggcac ctacatcaca gccgtaacag ccacatcaaa    240
   tgagatcaaa gagtcaccac tgcatgggac acaaaacacc atcaacaaga ggacccagcc    300
   cacctttggc ttcaccgtca attggaagtt ttcagagtcc accactgtct tcacgggcca    360
   gtgcttcata gacaggaatg ggaaggaggt cctgaagacc atgtggctgc tgcggtcaag    420
<210> 2
   <211> 540
   <212> DNA
   <213> Streptomyces avidinii
<400> 2
   ccctccgtcc ccgccgggca acaactaggg agtatttttc gtgtctcaca tgcgcaagat      60
   cgtcgttgca gccatcgccg tttccctgac cacggtctcg attacggcca gcgcttcggc     120
   agacccctcc aaggactcga aggcccaggt ctcggccgcc gaggccggca tcaccggcac     180
   ctggtacaac cagctcggct cgaccttcat cgtgaccgcg ggcgccgacg gcgccctgac     240
   cggaacctac gagtcggccg tcggcaacgc cgagagccgc tacgtcctga ccggtcgtta     300
   cgacagcgcc ccggccaccg acggcagcgg caccgccctc ggttggacgg tggcctggaa     360
   gaataactac cgcaacgccc actccgcgac cacgtggagc ggccagtacg tcggcggcgc     420
   cgaggcgagg atcaacaccc agtggctgct gacctccggc accaccgagg ccaacgcctg     480
   gaagtccacg ctggtcggcc acgacacctt caccaaggtg aagccgtccg ccgcctccat     540
<210> 3
   <211> 1335
   <212> DNA
   <213> Gallus gallus
<400> 3
   gaattctagg gacccgatcc acctgctggt gagtttgata ttcgtctctg gtcttcaatt     60
   ttggggttgt gcgttcaact ggaaaacgtg acccacccag attgcgtaac acctgggaag    120
   aaaagcctgc gccgggagca ataaaaggcg agggagcagg caggaggggt gagtcctgca    180
   aggagcacac ccggctgtcc acctgctgca gagatggtgc acgcaacctc cccgctgctg    240
   ctgctgctgc tgctcagcct ggctctggtg gctcctggcc tctctgccag aaaggtaatg    300
   gggtggggct gggagtgggt gcacctggtg cccacccctg cctcctgccc gccactgact    360
   ccttcttctt cgtcacagtg ctcgctgact gggaaatggg acaacgacct gggctccatc    420
   atgaccatcg gagctgtgaa cgacaatggc gagttcaatg gcacctacat cacagctgta    480
   gcagataatc caggaaacat cacgcgatca ccactgcttg ggatccaaca caaaagagcc    540
   tgccagccca cctttggctt cactgtccat tggaactttt caggtgcttc tctcccagcc    600
   tccctgcagt gtccctgctc ctctgctgtg cttccctgtg acaaacccct ttgctttcct    660
   gcccttcccc acgctgtctc cagtgctcgc ctgcccttcc ctacagactc cctgacggtc    720
   tctcctcctc actgtggtgt ccctgatgat ttccagccca tccctgcagt cccctcaaca    780
   atgccctgcc tcccatgccc ccggtgctgc cccatccctt cccgtagagc tgctgggctg    840
   ctgtcacctc ctggtccccg ggtgcagggg aggtgctggg gctgtcccca gagggcacgg    900
   agagctcaga tgagttgtcc cctgggcaga gggaccgtgg tgctggcact gccctgccct    960
   gcgtggggct cacaacccca ctcccctcat ctgccccttt tcccaacaga gtccaccagt   1020
   gtctttgtgg gccagtgctt cgtggacaag agtggaaagg aggtcctgaa gaccaaatgg   1080
   ctgcaacggt tagcagttga tgacattagt gatgactgga aagctaccag gtgagcccag   1140
   ggcagaggca cacggtccca ggctgtgact cgatggctgt gcacttccca ccttacatct   1200
   cctctctctc cccgcagggt cggcaacaac gacttcactc gccagcgcac agtggaggag   1260
   tgaggatggc cccgcaaagc cagcaacaat gccggagtgc tgacactgct tgtgatattc   1320
   ctcccaataa agctt                                                    1335
<210> 4
   <211> 1335
   <212> DNA
   <213> Gallus gallus
<400> 4
   gaattctaga gacccgatcc acctgctggt gagtttgata ttcgtctctg gtcttcaatt      60
   ttggggttgt gcgttcaact ggaaaacgtg acccacccag attgcgtaac acctgggaag     120
   aaaagcctgc gccgggagca ataaaaggcg agggagcagg caggaggggt gagtcctgca     180
   aggagcacac ccggctgtcc acctgctgca gagatggtgc acgcaacctc cccgctgctg     240
   ctgctgctgc tgctcagcct ggctctggtg gctcccagcc tctctgccag aaaggtaacg     300
   ggatggggct gggagtgggt gcacctggtg cccaccactg cctcctgccc gccactgact     360
   ccttcttctt cactgcagtg ctcgctgact ggggaatggg acaacgacct gggctccatc     420
   atgaccatcg gagctgtgaa cgacaatggc gagttcgatg gcacctacat cacagctgta     480
   gcagataatc caggaaacat cacgctatca ccactgcttg ggatccaaca caaaagagcc     540
   agccagccca cttttggctt cactgtccat tggaactttt caggtgcttc tctcccagcc     600
   tccctgcaat gtccctgctc ctctgctgtg cttccctgtg acaaacccct ctgctttcct     660
   gcccttcccc acgctgtctc cagtgctctc ctgcccttcc ctacagtctc cctgacggtc     720
   tctcctcctc gctgtggtgt ccctgatgat ttccagccct tccctgcaat cccctcaaca     780
   atgccctgcc tcccatgccc ccggtgctgc cccatccctt cccgtagagc tgctgggctg     840
   ctgtcacctc caggtccccg ggtgcagggg aagtgctggg gctgtcccca gagggcacag     900
   agagctcaga tgagttgtcc cctgggcaga gggaccatgg cactggcact gccctgccct     960
   gcgtggggct cacaacccca ctcccctcat ctgccccttt tcccaacaga gtccaccagt    1020
   gtctttgtgg gccagtgctt cgtggacagg agcggaaagg aggtcctgaa gaccaaatgg    1080
   ctgcaacggt tagcagttga tgacattagt gatgactgga tagctaccag gtgagcccag    1140
   ggcagaggca cacggtcccg ggctgtgact cgatggctgt gcacttccca ccttacatct    1200
   cctctctctc cccgcagggt cggcaacaac gacttcactc gccagcacac agtggaagag    1260
   tgaggatggc cccgcaaagc cagcaacaat gccagagtgc tgacactgct tgtgatattc    1320
   ctcccaataa agctt                                                     1335
<210> 5
   <211> 1133
   <212> DNA
   <213> Gallus gallus
<400> 5
   cctgctgcag agatggtgca cacaacctcc ccgctgctgc tgctgctgct gctcagcctg      60
   gctctggtgg ctcccagcct ctctgccaga aaggtaacgg gatggggctg ggagtgggtg     120
   cacctggtgc ccaccactgc ctcctgcccg ccactgactc cttcttcttc attgcagtgc     180
   tcgctgactg ggaaatggac caacaacctg ggctccatca tgaccatcag ggctgtgaac     240
   agcagaggtg aattcgcagg cacctacctc acagctgtag cagataatcc aggaaacatc     300
   aagctatcac cactgcttgg gatccaacac aaaagagcct gccagcccac ctttggcttc     360
   actgtccatt ggaacttttc aggtgcttct ctcccagcct ccctgcagtg tccctgctcc     420
   tctgctgtgc ttccctgtga caaacccctc tgctttcctg cccttcccca cgctgtctcc     480
   agtgctctcc tgcccttccc tacagtctcc ctgacggtct ctcctcctcg ctgtggtgtc     540
   cctgatgatt tccagctcat ccctgcaatc ccctcaacaa tgccctgcct cccatgcccc     600
   cggtgctgcc ccatcccttc ccgtagagct gctgggctgc tgtcacctcc tggtccccgg     660
   gtgcagggga ggtgctgggg ctgtccccag agggcacaga gagctcagat gagttgtccc     720
   ctgggcagag ggaccgtggt gctggcactg tcctgccctg cgtggggctc acgaccccac     780
   tcccctcatc tgcccctttt cccacagagt ccaccagtgt ctttgtgggc cagtgcttca     840
   tagacaggag cggaaaggag gtcctgaaga ccaaatggct gcaacggtta gcagttgatg     900
   acattagtga tgactggaaa gctaccaggt gagcccaggg cagaggcaca cggtcccggg     960
   ctgtgactcg atggctgtgc acttcccacc ttacatctcc tctctctccc cgcagggtcg    1020
   gctacaacaa cttcactcgc cagcgcacag tggaggagtg aggatggccc cgcaaagcca    1080
   gcaacaatgc cggagtgctg acactgcttg tgatattcct cccaataaag ctt           1133
<210> 6
   <211> 1334
   <212> DNA
   <213> Gallus gallus
<400> 6
   gaattctagg gacccgatcc acctgctggt gagtttgata ttcgtctctg gtcttcaatt      60
   ttggggttgt gcgttcaact ggaaaacgtg acccacccag attgcgtaac acctgggaag     120
   aaaagcctgc gccgggagca ataaaaggcg agggagcagg caggaggggt gagtcctgca     180
   aggagcacac ccggctgtcc acctgctgca gagatggtgc acacaacctc cccgctgctg     240
   ctgctgctgc tgctcagcct ggctctggtg gctcccagcc tctctgccag aaaggtaacg     300
   ggatggggct gggagtgggt gcacctggtg cccaccactg cctcctgccc gccactgact     360
   ccttcttctt cattgcagtg ctcgctgact gggaaatgga ccaacaacct gggctccatc     420
   atgaccatca gggctgtgaa cagcagaggt gaattcacag gcacctacct cacagctgta    480
   gcggataatc caggaaacat cacgctatca ccactgcttg ggatccaaca caaaagagcc    540
   agccagccca cctttggctt cactgtccat tggaactttt caggtgcttc tctcccagcc    600
   tccctgcagt gtccctgctc ctctgctgtg cttccctgtg acaaacccct ctgctttcct    660
   gcccttcccc acgctgtctc cagtgctctc ctgcccttcc ctacagtctc cctgacggtc    720
   tctcctcctc gctgtggtgt ccctgatgat ttccagccca tccctgcagt cccctcaaca    780
   atgccctgcc tcccatgccc ccggtgctgc cccatccctt cccgtagagc tgctgggctg    840
   ctgtcacctc caggtccccg ggtgcagggg aggtgctggg gctgtcccca gagggcacag    900
   agagctcaga tgagttgtcc cctgggcaga gggaccatgg cactggcact gccctgccct    960
   gcgtggggct cacaacccca ctcccctcat ctgccccttt tcccacagag tccaccactg   1020
   tcttcacggg ccagtgcttc atagacagga acgggaagga ggtcctgaag accatgtggc   1080
   tgctgcggtc aagtgttaat gacattagtt atgactggaa agctaccagg tgagcccagg   1140
   gcagaagcac acggtcccgg gctgtgactc aatggctgtg cacttcccac cttacatctc   1200
   ctcactctcc ccgcagggtc ggctacaaca acttcactcg cctgtgcaca gtggaggagt   1260
   gaggatggcc ccgcaaagcc agcaacaatg ccagagtgct gacactgctt gtgatattcc   1320
   tcccaataaa gctt                                                     1334
<210> 7
   <211> 1334
   <212> DNA
   <213> Gallus gallus
<400> 7
   gaattctaga gacccgatcc acctgctggt gagtttgata ttcgtctctg gtcttcaatt     60
   ttggggttgt gcgttcaact ggaaaacgtg acccacccag attgcgtaac acctgggaag    120
   aaaagcctgc gccgggagca ataaaaggcg agggagcagg caggaggggt gagtcctgca    180
   aggagcacac ccggctgtcc acctgctgca gagatggtgc acacaacctc cccgctgctg    240
   ctgctgctgc tgctcagcct ggctctggtg gctcccagcc tctctgccag aaaggtaacg    300
   ggatggggct gggagtgggt gcacctggtg cccaccactg cctcctgccc gccactgact    360
   ccttcttctt cattgcagtg ctcgctgact gggaaatgga ccaacaacct gggctccatc    420
   atgaccatca gggctgtgaa cagcagaggt gaattcacag gcacctacct cacagctgta    480
   gcggataatc caggaaacat cacgctatca ccactgcttg ggatccaaca caaaagagcc    540
   agccagccca cctttggctt cactgtccat tggaactttt caggtgcttc tctcccagcc    600
   tccctgcagt gtccctgctc ctctgctgtg cttccctgtg acaaacccct ctgctttcct    660
   gcccttcccc acgctgtctc cagtgctctc ctgcccttcc ctacagtctc cctgacggtc    720
   tctcctcctc gctgtggtgt ccctgatgat ttccagccca tccctgcagt cccctcaaca     780
   atgccctgcc tcccatgccc ccggtgctgc cccatccctt cccgtagagc tgctgggctg     840
   ctgtcacctc caggtccccg ggtgcagggg aggtgctggg gctgtcccca gagggcacag     900
   agagctcaga tgagttgtcc cctgggcaga gggaccatgg cactggcact gccctgccct     960
   gcgtggggct cacaacccca ctcccctcat ctgccccttt tcccacagag tccaccactg    1020
   tcttcacggg ccagtgcttc atagacagga acgggaagga ggtcctgaag accatgtggc    1080
   tgctgcggtc aagtgttaat gacattagtt atgactggaa agctaccagg tgagcccagg    1140
   gcagaagcac acggtcccgg gctgtgactc aatggctgtg cacttcccac cttacatctc    1200
   ctcactctcc ccgcagggtc ggctacaaca acttcactcg cctgtgcaca gtggaggagt    1260
   gaggatggcc ccgcaaagcc agcaacaatg ccagagtgct gacactgctt gtgatattcc    1320
   tcccaataaa gctt                                                      1334
<210> 8
   <211> 1133
   <212> DNA
   <213> Gallus gallus
<400> 8
   cctgctgcag agatggtgca cgcaacctcc ccgctgctgc tgctgctgct gctcagcctg      60
   gctctggtgg ctcccggcct ctctgccaga aaggtaacgg gatggggctg ggagtgggtg     120
   cacctggcgc ccacccctgc ctcctgcctg ccactgactc cttcttcttc gtcacagtgc     180
   tcgctgactg gggaatggga caacaacctg ggctccatca tgaccatcgg agctgtgaac     240
   gacaatggcg agttcaatgg cacctacatc acagctgtag cagataatcc aggaaacatc     300
   aagctatcac cactgcttgg gatccaacac aaaagagcct gccagcccac ctttggcttc     360
   actgtccatt ggaacttttc aggtgcttct ctcccagcct ccctgcaatg tccctgctcc     420
   tctgctgtgc ttccctgtga caaacccctc tgctttcctg cccttcccca cgctgtctcc     480
   agtgctctcc tgcccttccc tacagtctcc ctgacggtct ctcctcctcg ctgtggtgtc     540
   cctgatgatt tccagcccat ccctgcagtc ccctcaacaa tgccctgcct cccatgcccc     600
   cggtgctgcc ccatcccttc ccgtagagct gctgggctgc tgtcacctcc tggtccccgg     660
   gtgcagggga ggtgctgggg ctgtccccag agggcacgga gagctcagat gagttgtccc     720
   ctgggcagag ggaccgtggt gctggcactg ccctgccctg cgtggggctc acaaccccac     780
   tcccctcatc tgcccctttt cccacagagt ccaccagtgt ctttgtgggc cagtgcttcg     840
   tggacaggag cggaaaggag gtcctgaaga ccaaatggct gcaacggtta gcagttgatg     900
   acattagtga tgactggaaa gctaccaggt gagcccaggg cagaggcaca cggtcccggg     960
   ctgtgactcg atggctgtgc acttcccacc ttacatctcc tctctctccc cgcagggtcg    1020
   gctacaacaa cttcactcgc cagcgcacag tggaggagtg aggatggccc cgcaaagcca    1080
   gcaacaatgc cggagtgctg acactgcttg tgatattcct cccaataaag ctt 1133
<210> 9
   <211> 1133
   <212> DNA
   <213> Gallus gallus
<400> 9
   cctgctgcag agatggtgca cgcaacctcc ccgctgctgc tgctgctgct gctcagcctg     60
   gctctggtgg ctcccggcct ctctgccaga aaggtaacgg gatggggctg ggagtgggtg    120
   cacctggtgc ccacccctgc ctcctgcctg ccactgactc cttcttcttc gtcacagtgc    180
   tcgctgactg gggaatggga caacaacctg ggctccaaca tgaccatcgg agctgtgaac    240
   gacaatggcg agttcaatgg cacctacatc acagctgtag cagataatcc aggaaacatc    300
   aagctatcac cactgcttgg gatccaacac aaaagagcct gccagcccac ctttggcttc    360
   actgtccatt ggaacttttc aggtgcttct ctcccagcct ccctgcagtg tccctgctcc    420
   tctgctgtgc ttccctgtga caaacccctc tgctttcctg cccttcccca cgctgtctcc    480
   agtgctctcc tgcccttccc tacagtctcc ctgacggtct ctcctcctcg ctgtggtgtc    540
   cctgatgatt tccagctcat ccctgcaatc ccctcaacaa tgccctgcct cccatgcccc    600
   cggtgctgcc ccatcccttc ccgtagagct gctgggctgc tgtcacctcc tggtccccgg    660
   gtgcagggga ggtgctgggg ctgtccccag agggcacaga gagctcagat gagttgtccc    720
   ctgggcagag ggaccgtggt gctggcactg tcctgccctg cgtggggctc acgaccccac    780
   tcccctcatc tgcccctttt cccacagagt ccaccagtgt ctttgtgggc cagtgcttca    840
   tagacaggag cggaaaggag gtcctgaaga ccaaatggct gcaacggtta gcagttgatg    900
   acattagtga tgactggaaa gctaccaggt gagcccaggg cagaggcaca cggtcccggg    960
   ctgtgactcg atggctgtgc acttcccacc ttacatctcc tctctctccc cgcagggtcg   1020
   gctacaacaa cttcactcgc cagcgcacag tggaggagtg aggatggccc cgcaaagcca   1080
   gcaacaatgc cggagtgctg acactgcttg tgatattcct cccaataaag ctt          1133

## Description

### FIELD OF THE INVENTION

The present invention relates to new mutants of biotin binding proteins with improved properties compared to wild type proteins. Proteins, which bind biotin include chicken avidin and bacterial streptavidin, other poultry avidins, such as avidin proteins isolated from the duck, goose, ostrich and turkey, and chicken avidin-related proteins (AVRs).

### BACKGROUND OF THE INVENTION

Chicken avidin and bacterial streptavidin are widely utilized proteins in many life science applications ranging from purification techniques to modem diagnostics and targeted drug delivery. This methodology, known as (strept)avidin-biotin technology, relies on the extremely tight and specific affinity (Kd ~ 10⁻¹⁵ M) between (strept)avidin and biotin.

Avidin and streptavidin are exceptionally stable proteins consisting of homotetrameric up-and-down β-barrels, which upon biotin binding become even more stable. Transition midpoints of heat denaturation (Tₘ), analyzed by differential scanning calorimetry (DSC), have shown that avidin is more stable than streptavidin in both the absence and presence of biotin (Gonzalez, M., Argarana, C.E., & Fidelio, G.D., Biomol. Eng. 16, 67-72 (1999). A possible reason for this may be the intramonomeric disulphide bridge found in each avidin monomer. Wild-type (wt) avidin has a high isoelectric point and it is glycosylated, which properties may cause unspecific binding in some applications. Streptavidin is not glycosylated and lacks cysteine residues capable of forming disulfide bridges. It has been shown, that the unwanted properties of avidin can be abolished without markedly affecting the tight biotin-binding affinity and the stability characteristics of avidin (Marttila, A.T. et al., FEBS Lett 467 (2000), 31-6).The avidinand streptavidintetramer is actually a dimer of two dimers. The monomers that form the tetramer interact with each other in a symmetrical manner and form the three types of monomer-monomer interactions described in detail by Livnah (Livnah, O., Bayer, E.A., Wilcheck, M., & Sussman, J.L., Proceedings of The National Academy of Sciences of the United States of America 90 (1993), 5076-5080). The interaction between monomers 1-4 (and the equivalent 2-3) is the strongest, while the interaction between monomers 1-3 (and 2-4) is the weakest. The intensity of the interaction between monomers 1-2 (and 3-4) is also relatively weak, but important because tryptophan 110 in avidin (Trp 120 in streptavidin) from subunit 1 participates in biotin binding at the binding-site of subunit 2, forming the function-related monomer-monomer interface.

The 3-D structures of avidin and streptavidin have been solved by x-ray crystallography and their tertiary and quaternary structures show astonishing similarity despite relatively low amino acid sequence identity (Weber, P. C., Ohlendorf, D. H., Wendoloski, J. J. and Salemme, F. R. (1989) Science 243, 85-88; Livnah, O., Bayer, E. A., Wilcheck, M. and Sussman, J. L. (1993) Proc. Natl. Acad. Sci. 90, 5076-5080; Pugliese, L., Coda, A., Malcovati, M. and Bolognesi, M. (1993) J. Mol. Biol. 231, 698-710). Most of the essential biotin-binding residues are conserved, and the affinity for biotin is nearly identical in both avidin and streptavidin. In addition to high biotin-binding capacity, avidin and streptavidin tetramers show remarkable stability at high temperatures as well as under strongly denaturing conditions. The stability increases even more upon biotin binding (Green, M. (1990) Methods Enzymol. 184, 51-67; Gonzalez, M., Argarana, C. E. and Fidelio, G. D. (1999) Biomol. Eng. 16, 67-72).

A streptavidin mutant with enhanced stability characteristics when compared to those of the wild-type streptavidin has been reported previously (Reznik, G.O. et al., Nat Biotechnol 14 (1996), 1007-11). The improved stability was achieved by addition of two intermonomeric disulphide bridges to the streptavidin tetramer, one between monomers 1-3 and the other between monomers 2-4, by changing histidine residue 127 to cysteine. In avidin these interfaces are similar, and histidine residue 127 of streptavidin is analogous to isoleusine residue 117 of avidin.

The avidin gene (AVD) in chicken has homologues, called avidin-related genes (AVRs) (Keinänen, R. A., Laukkanen, M. L. and Kulomaa, M. S. (1988) J. Steroid Biochem. 30, 17-21;. Keinänen, R. A., Wallen, M. J., Kristo, P. A., Laukkanen, M. O., Toimela, T. A., Helenius, M. A. and Kulomaa, M. S. (1994) Eur. J. Biochem. 220, 615-21; Ahlroth, M. K., Kola, E. H., Ewald, D., Masabanda, J., Sazanov, A., Fries, R. and Kulomaa, M. S. (2000) Anim. Genet. 31, 367-75).

Seven different genes, AVR1-AVR7, have been cloned and sequenced. Two of the genes, AVR4 and AVR5, are 100 % identical in their coding sequence, exhibiting only a single nucleotide difference in their 5'-flanking region, whereas the others are 94-99 % identical to each other. The identity between AVD and the different AVRs ranges from 91 to 95 %. Messenger RNAs for AVR1, AVR2 and AVR3 have been detected in chicken under inflammatory conditions (Kunnas, T. A., Wallen, M. J. and Kulomaa, M. S. (1993) Biochim. Biophys. Acta 1216, 441-5), but it is not known whether they are expressed as proteins. The putative avidin-related proteins (AVRs), as deduced from their nucleotide sequences, are 74-81 % identical to avidin and 85-100 % identical to each other.

Avidin proteins, isolated from the duck, goose, ostrich and turkey, showed similar tetrameric structure, glycosylation and stability both against temperature and proteolytic activity of proteinase K as chicken avidin. The biotin-binding properties of these avidins, measured using IAsys optical biosensor, were similar to those found in avidin from the chicken. Three of these avidins, however, showed different immunological crossreactivity when compared to chicken avidin. The patient sera response to duck, goose and ostrich avidin was also lower than those observed for chicken and turkey avidins. These proteins could offer advantages over the use of chicken avidin and bacterial streptavidin in pretargeting applications (Hytönen et al., Biochem. J. (2003) 372; 219-225).

Because of the similarity of the structure of the biotin binding proteins including chicken avidin and bacterial streptavidin, other poultry avidins, such as avidin proteins isolated from the duck, goose, ostrich and turkey, and chicken avidin-related proteins (AVRs), it is presumed that new mutants of these biotin binding proteins may be created by site-directed mutagenesis of the amino acids of the sequences and that the resulting mutants have similar properties.

### SUMMARY OF THE INVENTION

The object of the present invention is to provide new mutants of biotin binding proteins with improved properties compared to wild type proteins. The term biotin binding proteins is understood to include chicken avidin and bacterial streptavidin, other poultry avidins, such as avidin proteins isolated from the duck, goose, ostrich and turkey, and chicken avidin-related proteins (AVRs).

This invention provides thermally stabilized biotin binding proteins using site-directed mutation. For avidin and AVRs this was achieved by introducing disulphide bridges between its subunits. These covalent bonds had no major effects on the biotin-binding properties of the respective mutants. Moreover, one of the mutants maintained its tetrameric integrity even in denaturing conditions. These new avidin forms have native → denatured transition midpoints (Tₘ) close to 100 °C in the absence of biotin; for AVR4/5 Tₘ is close to 110 °C. Furthermore, it is shown that the intramonomeric disulphide bridges found in wild-type avidin have effects on its stability.

A mutant form, in which this bridge was removed, had a lower Tm in the absence of biotin than wild-type avidin, but showed comparable stability in the presence of biotin.

In the present invention the stability of avidin was improved through the addition of intermonomeric disulphide bridges by changing isoleucine residue 117 to cysteine. In addition two supplementary mutants were produced where even more intermonomeric disulphide bridges were introduced. A cysteineless avidin version was also constructed, where the intramonomeric disulphide bridges of wild-type avidin were removed. The stability characteristics of these mutants were studied and compared with those of wild-type avidin and streptavidin.

Site-directed mutagenesis may also be introduced in other biotin binding proteins including bacterial streptavidin, other poultry avidins, and chicken avidin-related proteins (AVRs) in order to alter their properties similarly to avidin. The site-directed mutagenesis may also be a deletion of an amino acid.

The only AVR that exhibited affinity to biotin and 2-iminobiotin comparable to avidin was AVR4/5. Nonetheless, AVR4/5 showed other interesting differences, i.e. an "extra" cysteine residue in every subunit and a different glycosylation pattern, when compared with avidin.

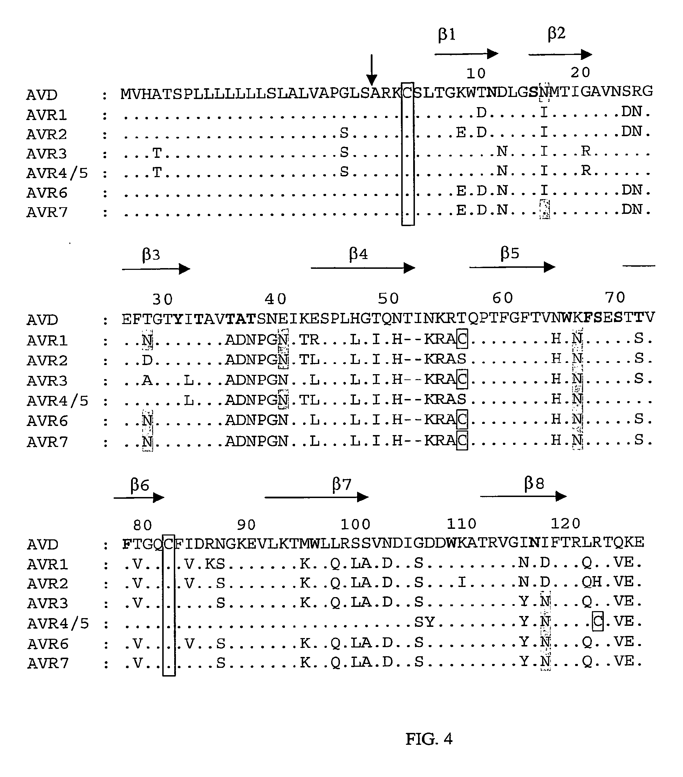

Site-directed mutagenesis of AVR4/5 cDNA was performed by mutation of the asparagine 43 of AVR4/5 to glutamic acid (AVR4/5(N43E)) in order to avoid possible glycosylation at that position. Cysteine 124 in AVR4/5 was converted to serine to eliminate the expected extraneous disulfide bridge in AVR4/5. The AVR4/5(C124S) was further mutagenized by changing the tyrosine 117 to cysteine (AVR4/5(Y117C)). The cysteine 124 in AVR4/5 could be converted to any amino acid. (The numbering of the amino acids are according to avidin (see figure 4)).

Suprisingly it was found that AVR4/5 displays the most durable heat stability characteristics of all biotin-binding proteins so far characterized. AVR4/5 exhibited a Tₘ that evidently exceeds the boiling point of water even in the absence of ligand. Due to its high thermal stability, AVR4/5 or some of its derivatives may prove extremely useful in applications where high heat durability is required.

### BRIEF DESCRIPTION OF THE FIGURES

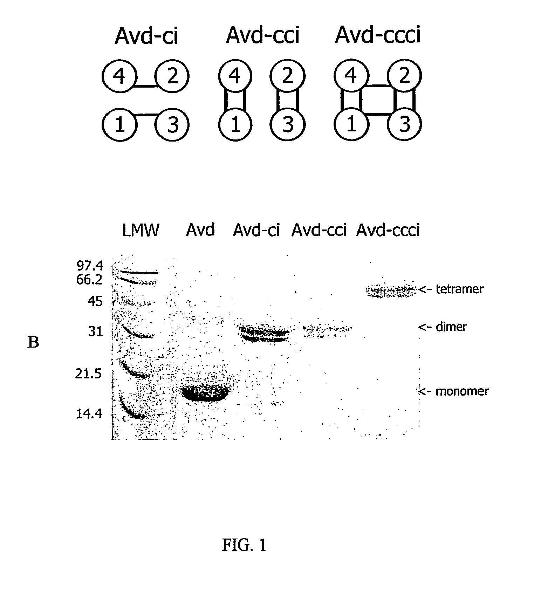

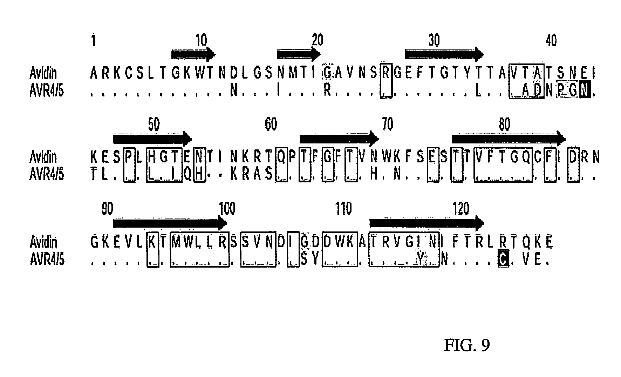

FIG. 1A shows schematic representation of the mutants Avd-ci, Avd-cci and Avd-ccci, displaying the intermonomeric disulphide bridges.
FIG. 1B shows non-reducing SDS-PAGE analysis of avidin (Avd) and avidin mutants Avd-ci, Avd-cci and Avd-ccci. Samples were boiled for 15 minutes in SDS-PAGE sample buffer (without β-mercaptoethanol) and the gel was stained with Comassie brilliant blue. Wild-type avidin (Avd) is mainly found as a monomeric form. The mutants that have intermonomeric disulphide bridges between two subunit pairs (Avd-ci, Avd-cci) and the mutant that forms a continuous macromolecule (Avd-ccci) formed dimeric and tetrameric structures.

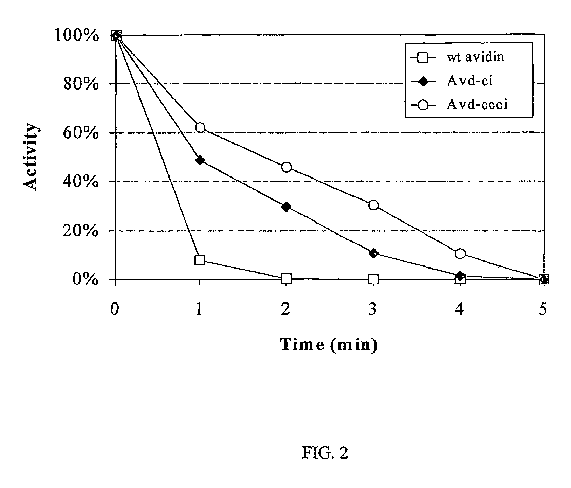

FIG. 2 shows the determination of binding of biotinylated alkaline phosphatase by wild-type avidin and the mutants Avd-ci and Avd-ccci after heat treatment (99.9 °C) for various periods of time by a microtiter plate assay.

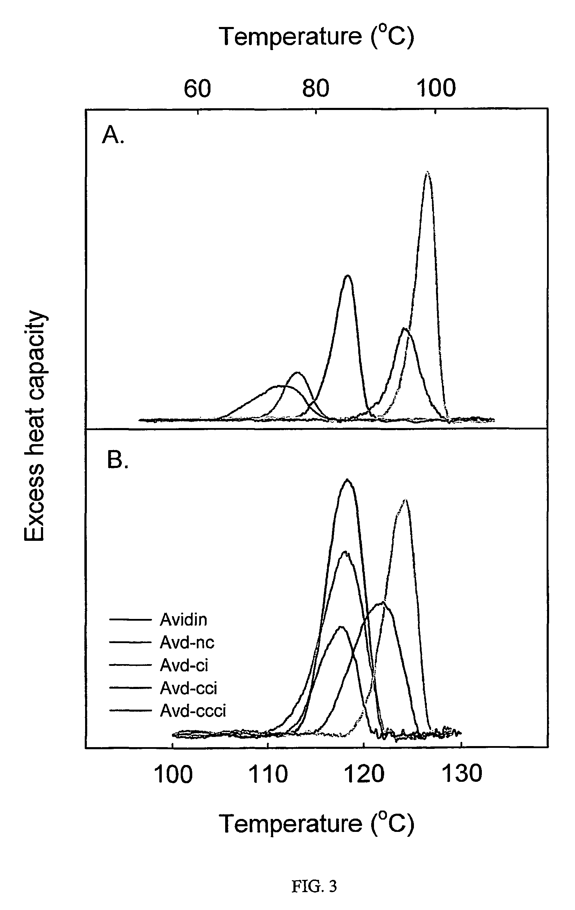

FIG. 3 is a representation of heat-induced unfolding of avidin and the mutants without (A) and with (B) biotin in a baseline subtracted form.
FIG. 4 is the sequence alignment of Avd and the AVRs (AVR1-AVR7). Dots indicate identical amino acids in AVRs as compared to avidin and the two amino acid deletion in AVRs is indicated by dashes. Horizontal arrows designate the β-sheets of avidin, and the vertical arrow indicates the cleavage site of the signal peptide in avidin. Biotin-binding residues are bold-faced in the avidin sequence, and the N-glycosylation site of avidin as well as the potential N-glycosylation sites of the AVRs are highlighted with gray. Cysteine residues are boxed.

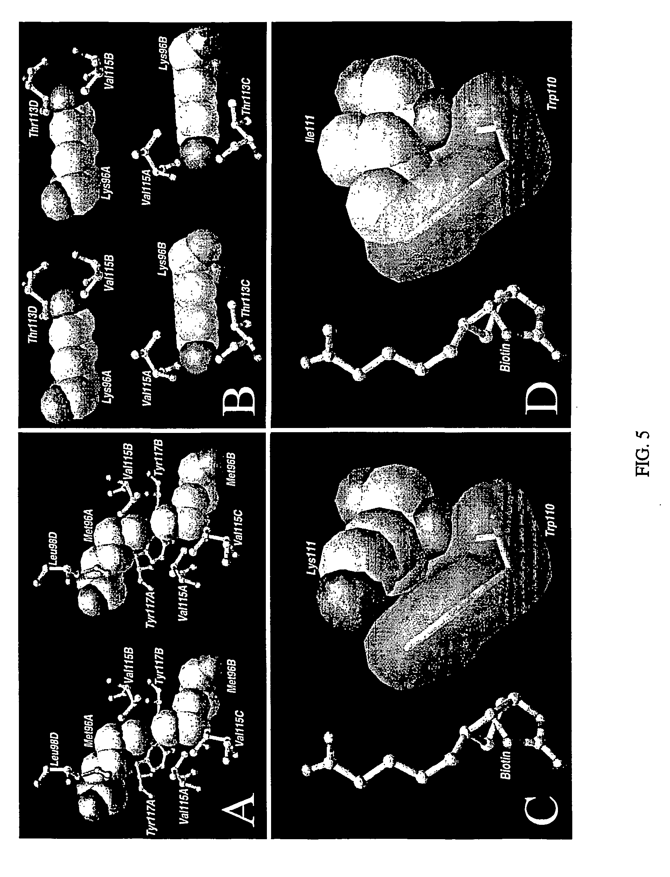

FIG 5. Re-organization of the subunit interface where sequence differences at position 96 are located (in stereo) and the effect of sequence differences at position 111 on the orientation of the side chain of Trp-110. The amino acid at position 96 is shown as a CPK-model and the surrounding amino acids are shown as ball-and-stick representations. a) Met-96 (avidin and AVR4/5), b) Lys-96 (AVR1-3,6,7). c) Lys-111 (avidin, AVR1-3-7), d) Ile-111 (AVR2) as CPK-models. Trp-110 is drawn as sticks along with the solvent accessible (transparent) surface; ball-and-stick models represent the ligand biotin. Note in d) the severe overlap between Ile-111 and Trp-110 that would force the reorientation of the tryptophan side chain, leading to poorer interactions with biotin and reduced binding affinity.

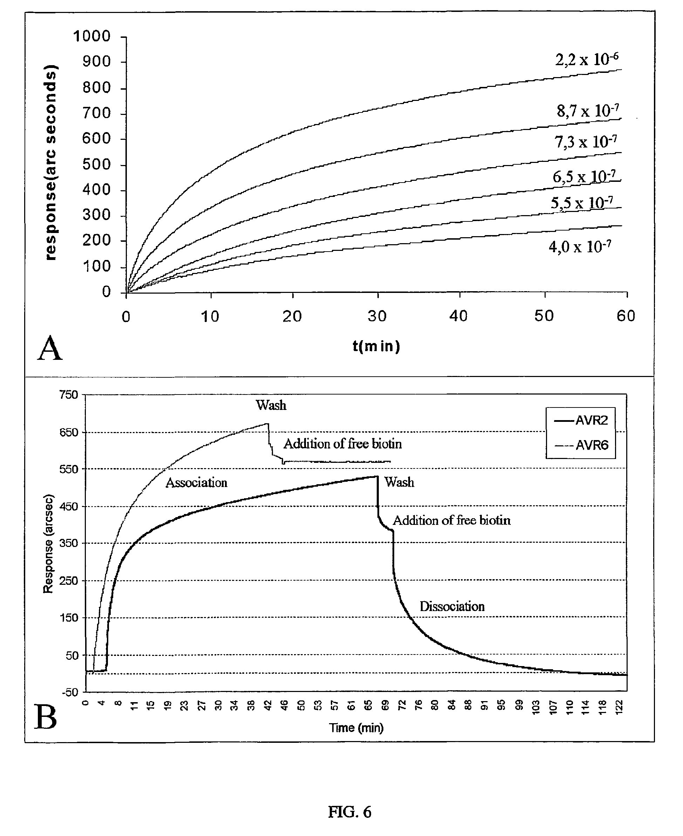

FIG 6. A) Binding curves for iminobiotin binding by AVR4/5 in different concentrations (in this case from 4 x 10-7 M to 2.2 x 10-6 M). The first 500 seconds were used to calculate the kass constant. Equilibrium state was obtained after approximately 1 hour measurement, depending on the concentration. The maximal binding (Rmax) for the highest concentration was ca. 1000 arc seconds. B) An example of IASyS reversibility experiment. The protein was allowed to bind a biotin-aminosilane cuvette. After reaching equilibrium, the cuvette was washed and dissociation was measured. A surplus of free biotin was then added, and the measurement was continued to achieve steady state. The cuvette was then washed again. The values measured after binding and dissociation, and after biotin treatment and second washing step, were used to define the reversibility of the biotin binding. In this particular experiment, the reversibility was practically 0 % for AVR6 and 100% for AVR2.

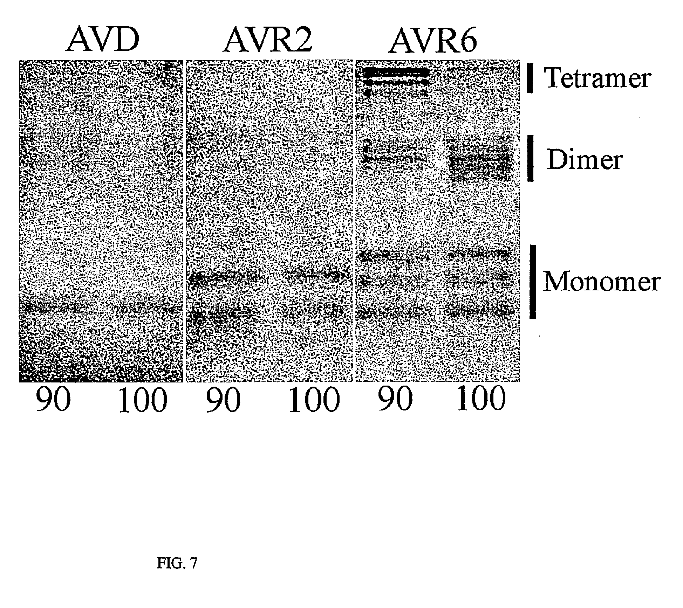

FIG 7. Non-reducing SDS-PAGE of avidin (AVD), AVR2 and AVR6 at 90°C and 100°C temperatures. The tetrameric, dimeric and monomeric forms are indicated with bars on the right side of the figure.

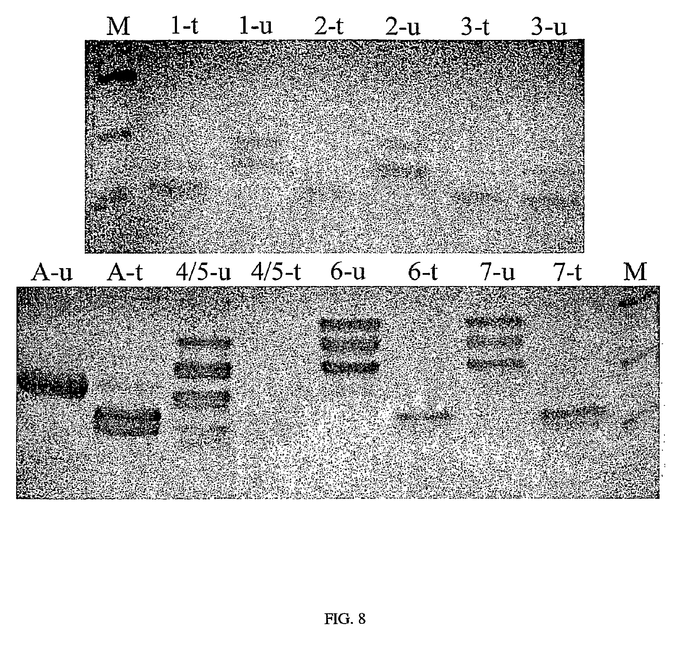

FIG 8. Glycosylation of avidin and AVRs in baculovirus-infected insect cells. Samples were either treated or non-treated with Endo Hf glycosidase followed by 15 % SDS-PAGE and staining with Coomassie Brilliant Blue. Avidin is denoted by A and the different AVRs by their corresponding numbers. M indicates the Biorad low molecular weight markers (31, 21.5 and 14.4 kDa).
   - u: Untreated with enzyme
   - t: Treated with enzyme
FIG 9. Sequence alignment of chicken avidin and AVR4/5. The secondary structure of chicken avidin is also shown (β-strands: arrows). Mutated amino acid residues are shaded with black. Amino acid residues located at the monomer-monomer interfaces are boxed. Potential differences explaining the high thermal stability of AVR4/5, in comparison to avidin, are shaded in gray.

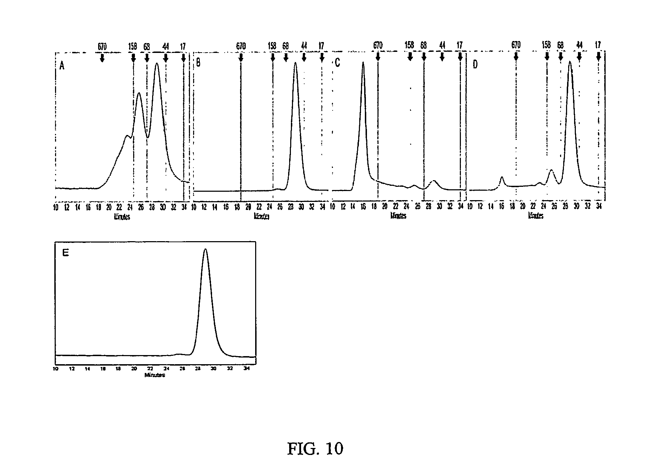

FIG 10. FPLC gel filtration chromatograms. A) AVR4/5 B) AVR4/5(C124S) C) Oxidized AVR4/5 D) AVR4/5 reduced with 2-mercaptoethanol E) AVR4/5(Y117C,C124S). Elution times of gel filtration standards were: Thyroglobulin (670 kDa) 18.8 min, IgG (158 kDa) 24.6 min, BSA (68 kDa) 27.1 min, Ovalbumin (44 kDa) 30.5 min, myoglobin (17 kDa), 33.9 min. Standard protein elution times are indicated with arrows in the chromatograms.

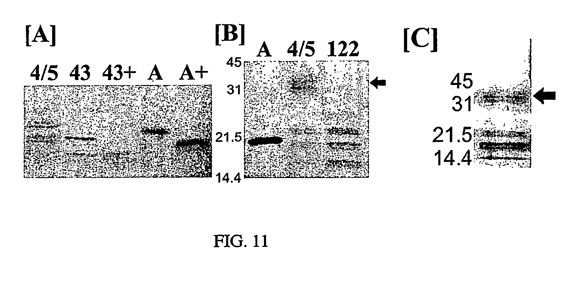

FIG 11. A) Deglycosylation analysis of the proteins. Denatured proteins treated with Endoglycosidase Hf analyzed with 15 % SDS-PAGE.
   4/5 = AVR4/5
   43 = AVR4/5(N43E)
   43+ = AVR4/5(N43E) treated with Endoglycosidase Hf
   A = avidin (Belovo)
   A+ = avidin treated with Endoglycosidase Hf

   B) Non-reducing SDS-PAGE. Samples were first acetylated in vitro, boiled for 20 minutes and subjected to 15 % SDS-PAGE. The arrow shows the presence of the dimeric form of AVR4/5. Values on the left represent bands for the molecular weight standards (Bio-Rad).
      A = avidin
      4/5 = AVR4/5
      124 = AVR4/5(C124S)
   C) Non-reducing SDS-PAGE. Samples were first acetylated in vitro, boiled for 20 minutes and subjected to 15 % SDS-PAGE. The arrow shows the presence of the dimeric form of AVR4/5(Y117C,C124S). Values on the left represent bands for the molecular weight standards (Bio-Rad).

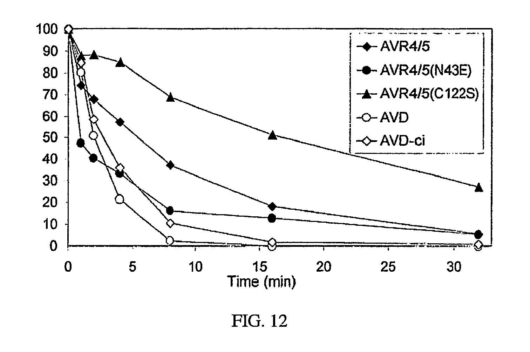

FIG 12. Microplate assay. AVR4/5 shows superior stability against boiling as compared to avidin as well as to high-stability AVD-ci (average, N=4).

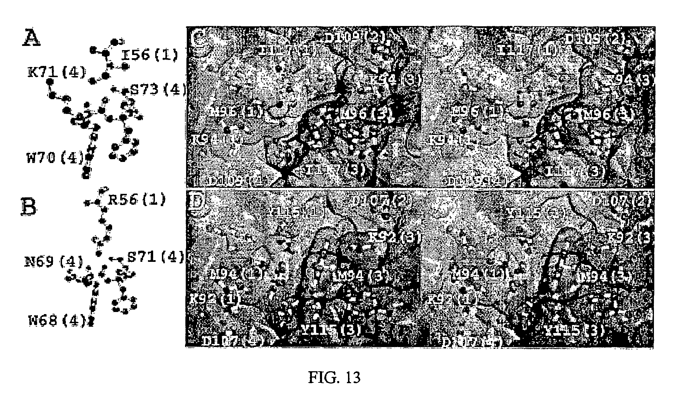

FIG 13. Sequence differences between avidin and AVR4/5 at the subunit interfaces. At the 1-4 (and 2-3) interface: A) I56 in avidin and B) R56 in AVR4/5. At the 1-3 (and 2-4) interface (in stereo): C) I117 in avidin and D) Y117 in AVR4/5. Interacting amino acids are shown as ball-and-stick representations. Subunit-surfaces are colored as follows: 1 = blue, 2 = yellow, 3 = red and 4 = green. In A) water molecules near the I117 seen in the crystal structure are shown as lone red spheres.

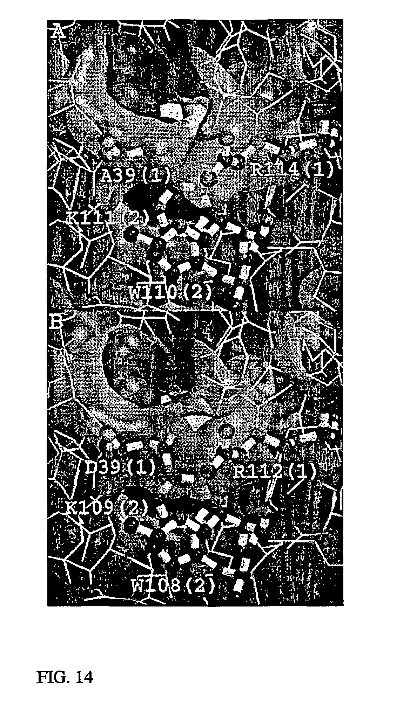

FIG 14. The salt bridge between D39 and R112 in AVR4/5. The potential salt bridge restricts the flexibility and size of the mouth of the ligand-binding cavity in comparison with avidin where alanine is present at position 39. The transparent surface is drawn for one AVR4/5 subunit, 1, but includes two interacting amino acids from subunit 2 of the AVR4/5 tetramer. Biotin is shown as a cpk-model, key amino acids as ball-and-stick figures, and other amino acids are represented by lines.

### DETAILED DESCRIPTION OF THE INVENTION

Avidin (SEQ ID NO 1) and streptavidin (SEQ ID NO 2) are valuable and widely used tools in the life sciences. In addition to their high biotin-binding affinity, the robustness and the flexibility of the system relies on their extreme stability under various demanding conditions. In the present application one object is to increase the stability of chicken avidin and other biotin binding proteins even further without losing its strong biotin-binding ability. In order to achieve this goal two, four or six intermonomeric disulphide bridges are introduced to avidin. The same could also be done to avidin related proteins. In addition, the role of the intramonomeric disulphide bridges in the stability of the avidin tetramer was determined, by removing them using site-directed mutagenesis.

According to the DSC results, both the intramonomeric and intermonomeric disulphide bridges have effects on the heat-induced denaturation of avidin. Removal of the intramonomeric disulphide bridges from avidin (Avd-nc) caused a decrease in its Tₘ in the absence of biotin (ΔTₘ = -8.9 °C), whereas in the presence of biotin the Tₘ was virtually the same as that of the wild-type avidin. This underlines the importance of the biotin-induced increase in the stability of avidin. Interestingly, the cysteineless mutant Avd-nc has a slightly higher Tm than that of streptavidin as an apoform and significantly higher as a complex with biotin. Further, avidin has naturally higher Tm than streptavidin.

The most stable of the mutated avidins is Avd-ci, which has an intermonomeric disulphide bridge in monomer interfaces 1-3 and 2-4. In contrast, the disulphide bridges created in interfaces 1-4 and 2-3 do not improve the thermal stability of avidin. Instead these mutations cause a decrease in the Tₘ of the mutant Avd-cci when compared to the wild-type avidin or even to the cysteineless mutant Avd-nc. This effect was more prominent in the absence of biotin. The high thermal stability of the combined mutant Avd-ccci indicated that the stabilizing effect of the disulphide bridge in interface 1-3 (and 2-4) exceeded the decrease caused by the disulphide bridges in interface 1-4 (and 2-3).

In the presence of denaturing agents it may be preferable to use the mutants Avd-ci, Avd-cci and Avd-ccci instead of wild-type avidin. This may particularly be the case for Avd-ccci since all of its monomers are covalently linked to each other (directly or indirectly). The mutant is capable of remaining as a tetramer even after unfolding, as seen in the SDS-PAGE analysis (Fig. 1B). Therefore it could be beneficial in applications where a leakage of subunits from matrix-coupled avidin tetramers would impede the qualitative and quantitative analysis of molecules. According to the DSC and microtiter plate assays, the avidin mutants Avd-ci and Avd-ccci, which had remarkably high Tₘ values even in the absence of biotin, could be utilized in PCR-protocols, because they can withstand the temperatures used to denature dsDNA. For example, the extraction of 2-iminobiotinylated or biotinylated ssDNA molecules at 95 °C is possible with Avd-ci and Avd-ccci coated particles.

In most applications of biotin binding technology high affinity biotin binding is essential. Consequently, all of the mutants introduced in this application are promising candidates for use with the methodology, as they all showed irreversible biotin binding and high affinity towards 2-iminobiotin. None of the mutations involved residues directly responsible for biotin binding. However, cysteine 106 that substituted isoleucine 106 in mutants Avd-cci and Avd-ccci, is located in the same loop (between β-strands 7 and 8) as the important biotin-binding residue tryptophan 110. The disulphide bridge formed between Cys106 of subunit 1 and Cys86 of subunit 4 may directly or indirectly have an influence on Trp110, and thereby also affect the biotin binding ability of these avidin mutants.

It was also found that the streptavidin tetramer is unstable in certain conditions without its bound ligand. This could be detrimental in many applications due to the loss of signal along with the analytical molecules. Furthermore, it would presumably shorten the life span of materials if they were to contain covalently linked streptavidin molecules. Chilkoti as well as Reznik have described a streptavidin mutant, His127Cys (Chilkoti, A., Schwartz, B. L., Smith, R. D., Long, C. J., and Stayton, P. S. (1995) Bio/Technology 13, 1198-1204; Reznik, G.O. et al., Nat Biotechnol 14 (1996), 1007-11). This mutant has an intermonomeric disulphide bridge between subunits 1 and 3 (2 and 4), which is analogous to the isoleucine 117 to cysteine substitution in our avidin mutant Avd-ci. They reported that the resultant mutant was more stable than wild type streptavidin (Reznik, G.O. et al., Nat Biotechnol 14 (1996), 1007-11. Avidin is, however, more stable than streptavidin, as judged by DSC analysis (Gonzalez, M. et al., Biomol Eng 16 (1999) 67-72). Therefore, the 1-3 (and 2-4) intermonomeric disulphide bridges bearing mutant Avd-ci would also have a higher Tm than the corresponding streptavidin mutant.

The production of streptavidin (or its mutants) is usually performed as inclusion bodies in E. coli. Its downstream processing includes laborious and time-consuming denaturation and renaturation procedures followed by the contrived formation of disulphide bridges (as in the case of the His127Cys mutant) and additional purification steps. In contrast, according to the present invention, the production of avidin and other biotin binding mutants, even those with intermonomeric disulphide bridges, yielded soluble proteins in insect cells that were easily purified in a single step by affinity chromatography on a 2-iminobiotin column.

The avidin mutants and other avidin related protein mutants include a signal sequence and thus are introduced in a eucaryotic insect cell to a special path, where building up of the multiple disulphide bridges is possible as part of the process. The mutants are thus soluble and functional immediately after this process. In this special path the bridging is enhanced by i.a. PDI (Protein Disulphide Isomerase) enzymes. On the contrary the streptavidin mutants are produced in an inactive form in procaryotic bacteria (US patent 6,022,951). Streptavidin has not been succesfully produced to a special path in an insect cell/baculovirus system, which system has now successfully been used in avidin and avidin mutant production. Avidin also has considerably higher solubility than streptavidin. Previously it was thought that because avidin already has one natural intramonomeric disulfide bridge, introducing new intermonomeric disulphide bridges would cause incorrect pairing leading to low quality or inactive protein in the cells. Streptavidin does not have a natural cysteine and thus this would not be a problem in streptavidin production.

Avidin and streptavidin are totally different proteins even though they have a similar structure. The mutant avidin Avd-ccci differs from the known avidins in that all the subunits are covalently attached to each other. It includes in addition to I117C also changes where intermonomeric bridges are formed by amino acids in different positions of the subunits, i.e. cysteine 86 pairs with cysteine 106 and vice versa (1-4 monomers and 2-3 monomers) and thus differs from the I117C mutant (or H127C streptavidin mutant), wherein the same amino acid in the pairing subunits are bridged in the disulphide bridging. Several disulphide bridges have not been introduced in a streptavidin tetramer than could be achieved by H127C, and such streptavidin mutant could be non-functional and presumably very heterogeneous. When the side chains of isoleucines 117 in each avidin tetramer subunit are substituted with cysteines they form the 1-3 (and 2-4) disulphide bridges in mutants Avd-ci and Avd-ccci. Each subunit has isoleucine 106 and aspartate 86, in mutants Avd-cci and Avd-ccci these are substituted with cysteines in order to form 1-4 (and 2-3) intermonomeric disulphide bridges.

The gene encoding avidin (AVD) has seven homologues in chicken, named avidin-related genes (AVRs, AVR1-AVR7 (SEQ ID NOs 3-9)). Since it was not known if the avidin-related or *AVR* genes, are expressed as proteins in chicken, their cDNAs were recently cloned and they were produced using a baculovirus-insect cell expression system (Laitinen, O. H., Hytönen, V. P., Ahlroth, M. K., Pentikäinen, O. T., Gallagher, C., Nordlund, H. R., Ovod, V., Marttila, A. T., Porkka, E., Heino, S., Johnson, M. S., Airenne, K. J. & Kulomaa, M. S. (2002) Biochem. J. 363, 609-617) Two of the genes, AVR4 and AVR5, are 100 % identical in their coding sequence, exhibiting only a single nucleotide difference in their 5'-flanking region, whereas the others are 94-99 % identical to each other. The identity between AVD and the different AVRs ranges from 91 to 95 % (Keinänen, R. A., Wallen, M. J., Kristo, P. A., Laukkanen, M. O., Toimela, T. A., Helenius, M. A. and Kulomaa, M. S. (1994) Eur. J. Biochem. 220, 615-21; Ahlroth, M. K., Kola, E. H., Ewald, D., Masabanda, J., Sazanov, A., Fries, R. and Kulomaa, M. S. (2000) Anim. Genet. 31, 367-75). The putative avidin-related proteins (AVRs), as deduced from their nucleotide sequences, are 74-81 % identical to avidin and 85-100 % identical to each other.

Amino acid sequence analysis and molecular modelling are used to predict and explain the properties of the AVRs. It was found that the AVR proteins are highly similar to avidin both structurally and functionally. Despite the numerous amino acid substitutions in the subunit interface regions, the AVRs form extremely stable tetramers similar to avidin.

Throughout the present application the sequences of avidin and AVRs are aligned so that the amino acids 55 (threonine) and 56 (isoleucine) of the avidin sequence which do not exist in the sequence of the AVRs, are counted in the AVR sequence (see figure 4). Thus for example the asparagines 117 in the AVRs AVR1 and AVR2 correspond to the isoleucine 117 in avidin, while the number of the amino acid 117 in AVRs would be 115 if the two amino acids 55 and 56 were not counted in the sequence.

Differences are found in some physico-chemical properties of the AVRs as compared to avidin, including lowered isoelectric point, increased glycosylation and, most notably, reversible biotin binding for two AVRs (AVR1 and AVR2). Molecular modelling shows how the replacement Lys-111-Ile in AVR2 alters the shape of the biotin-binding pocket and thus results in reversible binding. Biochemical analyses show that disulfide bonds can form and link monomers in AVR4/5, a property not found in avidin.

Despite the relatively high primary sequence conservation (figure 4), there are interesting amino acid substitutions that make the physico-chemical properties of AVRs different from those of avidin. For example, the isoelectric point of some AVRs is neutral or even acidic, in contrast to basic for avidin, and their glycosylation patterns also differ. Furthermore, differences in biotin binding is observed, regardless of the fact that almost all of the amino acid residues important for biotin binding in avidin and streptavidin are conserved in the AVRs. In previous studies, the AVR sequences have been used as models for lowering the pI of avidin (Marttila, A. T., Airenne, K. J., Laitinen, O. H., Kulik, T., Bayer, E.., Wilchek, M. and Kulomaa, M. S. (1998) FEBS Lett. 441, 313-317) and to produce non-glycosylated avidin (Marttila, A. T., Laitinen, O. H., Airenne, K. J., Kulik, T., Bayer, E. A., Wilchek, M. and Kulomaa, M. S. (2000) FEBS Lett. 467, 31-6.)

All of the pI-mutants, as well as the non-glycosylated avidins, bind biotin with high affinity and form stable tetramers. Therefore, differences in pI or the lack of glycosylation at positions corresponding to residue 17 in avidin is not expected to affect biotin binding and tetramer formation of the AVRs. Consequently, the observed differences must be based on yet unknown features that, while affecting biotin binding, do not impede tetramer stability.

Another structurally interesting feature of the AVRs (except for AVR2) is the third cysteine residue. In addition to AVR4/5, this may at least partially explain the properties of AVRs 1, 3, 6 and 7. Their inability to bind 2-iminobiotin in IASyS analyses may reflect the presence of such structural differences as compared to avidin.

In conclusion, the recombinant AVRs are functional and show interesting physico-chemical properties. These new biotin binders may provide advantages over avidin and streptavidin in several applications. For example, AVR2 could be used in affinity purification protocols that require mild elution conditions.

Further because of the similarity of the sequences of avidin and AVRs, the site-directed mutations performed to the avidin sequence in order to create thermostabile mutant avidin may be performed in a similar way for the AVRs thus creating intermonomeric disulphide bridges in the tetramer. The tetramer may have one disulphide bridge between monomers 1-3 and one disulphide bridge between monomers 2-4 as in Avd-ci, two disulphide bridges between monomers 1-4 and two disulphide bridges between monomers 2-3 as in Avd-cci, or one disulphide bridge between monomers 1-3 and one disulphide bridge between monomers 2-4 and two disulphide bridges between monomers 1-4 and two disulphide bridges between monomers 2-3 as in Avd-ccci.

The AVRs AVR1 and AVR2 have asparagine 117 instead of the isoleucine 117 in avidin, which is changed to cysteine in these mutants, and in AVR3-7 the amino acid residue 117 is tyrosine, which is changed to cysteine. The isoleucine residue 106 and aspartate residue 86 are the same in the avidin and the AVRs (see figure 4).

AVR4/5 is the closest relative to the avidin gene in the family, and its chromosomal location is closest to the avidin gene (Ahlroth, M. K., Grapputo, A., Laitinen, O. H. & Kulomaa, M. S. (2001) Biochem. Biophys. Res. Commun. 285, 734-741). It is the only AVR that exhibited affinity to biotin and 2-iminobiotin comparable to avidin. The most obvious structural differences would arise from the diverse patterns of glycosylation, and the extraneous cysteine residue found in most AVRs that is not present in avidin. In addition, residues in contact at the subunit interfaces of the model structure showed significant differences when compared to the avidin structure. However, according to the analysis of stability by SDS-PAGE (Bayer, E. A., Ehrlich-Rogozinski, S. & Wilchek, M. (1996) Electrophoresis 17, 1319-1324), these differences at the interfaces did not reduce the stability of the AVRs.

In the present study, we have carried out a more detailed examination of the structural and stability properties of the AVR4/5 protein using comprehensive biochemical and mutational analyses. A surprising observation of the present invention was the superior heat stability of AVR4/5 and its mutants when compared to avidin or streptavidin (Gonzalez, M., Argarana, C. E. & Fidelio, G. D. (1999) Biomol. Eng. 16, 67-72), and to the thermally improved avidin forms, too (Nordlund, H. R., Laitinen, O. H., Uotila, S. T., Nyholm, T., Hytönen, V. P., Slotte, J. P. & Kulomaa, M. S. (2003) J. Biol. Chem. 278, 2479-2483). In the latter case, the improvements in the stability were achieved by introducing disulfide bridges between avidin subunits. In contrast, the disulfide bridge due to the extraneous Cys-124 of AVR4/5 is not connected to the enhanced stability, since AVR4/5(C124S) was observed to be even more stable than AVR4/5. According to ITC, DSC and optical biosensor data, the biotin and 2-iminobiotin binding properties of AVR4/5 resemble those of avidin. The measured affinity towards biotin (K_{d} ≈3.6 × 10⁻¹⁴ M) is weaker than that for avidin, but this interaction is still one of the tightest measured for ligand-protein-interactions. It actually resembles values measured between biotin and bacterial streptavidin (K_{d} ≈4.0 × 10⁻¹⁴ M) (Green, N. M. (1990) Methods Enzymol. 184, 51-67). The thermodynamic explanation for the weaker affinity of AVR4/5 towards biotin (as compared to avidin) seems to lie in the entropy change on binding. Due to its high thermal stability, AVR4/5 or some of its derivatives may prove extremely useful in applications where high heat durability is required.

The modeling results suggest that possible reasons for the improved thermal stability include both a more stable tertiary structure of the AVR4/5 β-barrel as well as more favorable subunit interactions of the tetramer. The structure of avidin may be more flexible and in a more open conformation to accept biotin entry into the binding pocket than the potentially more rigid AVR4/5 barrel. This might also explain the higher affinity of avidin towards biotin and 2-iminobiotin. The potential salt bridge between Asp-39 and Arg-114 in AVR4/5 could contribute to the observed slower association rate of AVR4/5 for 2-iminobiotin, and it may also stabilize the monomer structure. Furthermore, the interaction of Asp-39 with the positively charged Lys-111 from the neighboring subunit may enhance the stability of the tetramer. Another contribution to the slower association rate may be the presence of the Pro-41-Gly-42 dipeptide in AVR4/5 that could form a conformation that partially blocks entry of biotin into the binding-pocket.

One important factor for the enhanced stability is probably connected to the deletion of two residues in loop 4 of AVR4/5, corresponding to residues Thr-55 and Ile-56 in avidin. This deletion may eliminate the unfavorable interaction seen in avidin. In general, comparison of the crystal structure of related proteins have shown that shorter loops are less mobile and presumably more stable (Usher, K. C., de la Cruz, A. F., Dahlquist, F. W., Swanson, R. V., Simon, M. I. & Remington, S. J. (1998) Protein Sci. 7, 403-412; Davail, S., Feller, G., Narinx, E. & Gerday, C. (1994) J. Biol. Chem. 269, 17448-17453; Thompson, M. J. & Eisenberg, D. (1999) J. Mol. Biol. 290, 595-604). In addition, the Ser-41 to proline conversion in loop 3 may add rigidity to the secondary and tertiary structure of AVR4/5. The replacement in AVR4/5 of glycine residues, present in avidin, may have a further stabilizing effect, since glycine is potentially more mobile and has more freedom to adopt torsion angles not permitted for other amino acids (Liu, H. L., Doleyres, Y., Coutinho, P. M., Ford, C. & Reilly, P. J. (2000) Protein Eng. 13, 655-659; Shibuya, H., Kaneko, S. & Hayashi, K. (2000) Biochem. J. 349, 651-656).

Finally, the replacement of Ile-117 in avidin by tyrosine in AVR4/5 should increase the stability of the 1-3 (and 2-4) dimer interface in comparison with avidin. Furthermore, the stabilizing effect might also extend to subunits 2 and 4 (1 and 3), which are in close proximity to the 1-3 (2-4) dimer interface (Figure 13D). Additional mutational and structural analyses are, however, needed to validate the accuracy of these proposals.

Although the predicted disulfide bridges seemed to have no effect on the thermal stability of AVR4/5, they had a clear influence on the overall structure of the protein. In contrast to the previous hypothesis (Laitinen, O. H., Hytönen, V. P., Ahlroth, M. K., Pentikäinen, O. T., Gallagher, C., Nordlund, H. R., Ovod, V., Marttila, A. T., Porkka, E., Heino, S., Johnson, M. S., Airenne, K. J. & Kulomaa, M. S. (2002) Biochem. J. 363, 609-617), the FPLC data suggested that these disulfide bridges connect AVR4/5 tetramers together, thus forming larger oligo-tetrameric structures instead of linking the monomers within the same tetramer (Figure 10). Multimerization has direct consequences on the possible applications where AVR4/5 could be exploited. In some cases where high thermal stability connected to a clearly defined molecular mass is needed, then AVR4/5(C124S) or its derivatives could be the best option. In other circumstances, for example where high biotin-binding capacity together with high thermostability is required (Välimaa, L., Pettersson, K., Vehniäinen, M., Karp, M. & Lövgren, T. (2003) Bioconjug. Chem. 14, 103-111), native AVR4/5 might be the most suitable candidate.

Based on the sequence, AVR4/5 contains three potential N-glycolysation sites. In a previous study it seemed that AVR4/5 was heavily glycosylated, but it was impossible to judge if all possible sites were utilized (Laitinen, O. H., Hytönen, V. P., Ahlroth, M. K., Pentikäinen, O. T., Gallagher, C., Nordlund, H. R., Ovod, V., Marttila, A. T., Porkka, E., Heino, S., Johnson, M. S., Airenne, K. J. & Kulomaa, M. S. (2002) Biochem. J. 363, 609-617). Mutational analysis showed that glycosylation occurs at least at Asn-43 (Figure 11). However, the glycosylation of Asn-43 had no marked influence on biotin binding, thermal stability or structural properties of AVR4/5. A portion of the resultant protein AVR4/5(N43E) appeared to be even more heavily glycosylated than avidin suggesting that both remaining glycolysation sites (Asn-71 and Asn-119) could be utilized at least to some extent.

By constructing and analyzing the AVR4/5(Y117C,C124S), it was shown that it is possible to apply the mutation(s) described in (Nordlund, H. R., Laitinen, O. H., Uotila, S. T., Nyholm, T., Hytönen, V. P., Slotte, J. P. & Kulomaa, M. S. (2003) J. Biol. Chem. 278, 2479-2483) to AVR4/5(C124S). The obtained protein AVR4/5(Y117C,C124S) is a stable tetramer including covalent bonds between subunits. This kind of protein could offer even better properties in applications, where high resistance against environmental factors is needed. One potential use may be, for example, in surface-based applications, where biotin-binding protein is attached to a matrix and the subunit dissociation is harmful event.

The behaviour of AVR4/5-protein was changed significantly due to mutation Cys124 -> Ser. AVR4/5 forms oligomers of tetramers via intertetrameric disulphide bonds. AVR4/5(C124S) behave like a native avidin with the high stability of AVR4/5. The obtained protein AVR4/5(C124S) was found to be even more stable than original one (AVR4/5). This protein (AVR4/5(C124S)) may found to be useful in many applications in avidin-biotin technology.

It is shown in this application that the intramonomeric disulphide bridges of wild-type avidin seem to be an important factor in making avidin so thermostable. On the other hand, it is possible to enhance the high thermostability of avidin and other biotin binding proteins further by the introduction of intermonomeric disulphide bridges. The most thermostable avidin mutants and other biotin binding protein mutants described in this application provide more stable tools for biotin binding technology, suitable also for new kinds of applications.

The invention will be further described with reference to the following non-limiting examples.

### EXAMPLES

### Example 1.

### Design of the avidin mutants

Mutations were designed by using the sequence and structure information obtained from analyses with GCG (Genetics Computer Group, Madison, Wisconsin), EMBOSS (European Molecular Biology Open Software Suite), WHAT IF (Vriend, G., J. Mol. Graph. 8 (1990), 52-6, 29) and InsightII (Molecular Simulations Inc., San Diego, CA) programs. Genetic engineering of the coding sequence of avidin was performed by megaprimer (Sarkar, G. & Sommer, S.S., Biotechniques 8 (1990), 404-407) and QuikChange (Stratagene) methods, by using oligonucleotide primers containing the desired mutations.

The avidin mutant Avd-nc (C4A, C83Y) was constructed to obtain information about the importance of the intrinsic disulphide bridges to the overall stability of the avidin tetramer. According to the sequence alignment with streptavidin, the cysteine residues were substituted with the same residues that streptavidin bears in the analogous positions in its primary structure (Table 1). The avidin mutant Avd-cci (D86C, I106C) adopted also the cysteines using the evolutionary approach (Laitinen, O.H. et al., FEBS Lett 461 (1999), 52-8). The sequence information came from the avidin-like domain of the sea urchin fibropellins (Hunt, L.T. & Barker, W.C., Faseb 3 (1989), 1760-1764; Delgadillo-Reynoso, M.G. et al., J Mol Evol 29 (1989), 314-27; Bisgrove, B.W. et al., Journal of Molecular Evolution 41 (1995), 34-45; Bisgrove, B.W. & Raff, R.A., Dev Biol 157 (1993), 526-38. In the case of Avd-cci, two intermonomeric disulphide bridges, designed to form between monomers 1-4 and 2-3 were introduced, thereby constituting a total of four new disulphide bridges per tetramer (Fig. 1A). In Avd-cci cysteine 86 from subunit 1 was presumed to pair with cysteine 106 from the adjacent subunit 4 and vice versa. Identical contacts were assumed to be present on the interface of subunits 2 and 3 as well.

**Table 1. Description of the proteins. Number of intermonomeric disulphide bridges and the measured affinity towards 2-iminobiotin are indicated. Superscript (*) denotes that Avd-nc has no cysteines due to the removal of the wild-type intramonomeric disulphide bridge.**

| Sample | Mutated residues | No. of intermonomeric disulphide bridges | 2-iminobiotin affinity K_{d} (M) |
|---|---|---|---|
| wt Avidin | None | 0 | 2.5 x 10⁻⁸ |
| Avd-nc | C4A, C83Y | 0* | 1.9 x 10⁻⁷ |
| Avd-ci | I117C | 2 | 8.5 x 10⁻⁸ |
| Avd-cci | D86C, I106C | 4 | 9.4 x 10⁻⁸ |
| Avd-ccci | D86C, I106C, | 6 | 2.9 x 10⁻⁷ |
| | I117C | | |

Avd-ci (I117C) has an extra cysteine residue in each subunit of the tetramer. It was designed according to the mutational strategy that Chilkoti (Chilkoti, A. et al., Bio/Technology 13 (1995), 1198-1204) and Reznik (Reznik, G.O. et al., Nat Biotechnol 14 (1996), 1007-11) used to stabilize the streptavidin tetramer. In Avd-ci, cysteine 117 from subunit 1 faces cysteine 117 from the neighboring subunit 3. Identically, the corresponding subunit interface 2-4 of the wild-type protein contains two equivalent isoleucine residues, which were replaced by cysteines. Therefore, two intermonomeric disulphide bridges in the avidin tetramer were expected to form between subunits 1-3 and 2-4, intensifying the firm association between dimers 1-4 and 2-3 by the addition of two covalent bonds. Finally, in order to introduce six intermonomeric disulphide bridges into the avidin tetramer, Avd-ccci, a combination of mutants Avd-ci and Avd-cci, was constructed.

### Example 2.

### Production, purification and characterization of mutant avidins

All the mutants were produced by a baculovirus expression system (Bac-To-Bac, Gibco BRL, Life Technologies, Gaithersburg, MD, USA) in the infected insect cells and purified by affinity chromatography on 2-iminobiotin agarose as previously described in detail by Airenne (Airenne, K.J. et al., Protein Expression and Purification 9(1997), 100-108) and Laitinen (Laitinen, O.H. et al., Biochem J. 363 (2002), 609-17).

Wild-type avidin was purified from chicken egg-white. Using a Vibra cellTM sonicator, the egg-white was sonicated for 3 minutes on ice at power setting 8 and 50 % duty cycle with a one-minute break between bursts. After sonication the sample was diluted with two volumes of PBS and centrifuged (20 minutes, 20.000 g, 4 °C). The soluble fraction was further purified by affinity chromatography on 2-iminobiotin agarose as previously reported (Laitinen, O.H. et al., J Biol Chem 276 (2001), 8219-24). Protein samples were concentrated and subjected to change of buffers with Centricon YM-3 filters (Millipore, cat. no. 4202). The SDS-PAGE analysis was performed with a sample buffer without β-mercaptoethanol.

All the mutants showed excellent purification efficiency, indicating that the mutations had no major effects on the 2-iminobiotin-binding properties. Moreover, the mutants showed irreversible biotin-binding properties indistinguishable from that of wild-type avidin as measured with an IAsys optical biosensor (data not shown). Affinities towards 2-iminobiotin were determined for wild-type avidin and the four mutants (Table 1). The results indicated that the mutations had not significantly altered the 2-iminobiotin binding-characteristics of the mutants. The formation of the intermonomeric disulphide bridges was studied with SDS-PAGE analysis (Fig. 1B), which confirmed that the cysteines formed pairs in the manner expected. The biotin-binding activity after heat treatment of Avd-ci and Avd-ccci was studied by a microtiter plate assay (Fig. 2). The mutants proved to be more stable and they remained active, in the sense of binding biotin, longer than the wild-type avidin.

### Example 3.

### Biotin-binding assays for avidin and the mutants

Reversibility of biotin binding was measured for avidin and the mutants with an IAsys optical biosensor (Laitinen, O.H. et al., FEBS Lett 461 (1999), 52-8). Protein samples were allowed to bind to a biotin-aminosilane cuvette in PBS containing 1 M NaCl. After the equilibrium was reached, biotin-containing buffer was added and the dissociation of the proteins was monitored. The affinities of the proteins towards 2-iminobiotin were determined with an IAsys optical biosensor (Marttila, A.T. et al. FEBS Lett 441 (1998), 313-7). Biotin-binding activity of the mutants Avd-ci and Avd-ccci after heat treatment was studied with a microtiter plate assay. Protein samples, 5 µg / ml concentration in PBS, were heated for various time periods at 99.9 °C and then chilled on ice. The samples were transferred to a Nunc Maxisorp-plate and incubated at 37 °C for 2 h. The wells were washed three times with PBS-Tween (0.05 % v / v). After that the wells were blocked with PBS containing 1% BSA at 37 °C for 30 minutes and washed again with PBS-Tween. Next, biotinylated alkaline phosphatase (Sigma) in PBS, 1% BSA was added and incubated at 37 °C for one hour. Once again, the wells were washed with PBS-Tween and the PNPP substrate in DEA buffer (1 mg / ml) was applied to the wells. The absorbances at 405 nm were measured after incubation for 45 minutes.

### Example 4.

### Differential scanning calorimetry of the avidin mutants

In order to study the thermal stability of the purified avidin mutants, they were subjected to analysis by differential scanning calorimetry. A Nano II differential scanning calorimeter (Calorimetric Science Corporation, Provo, Utah) was used. The protein sample concentrations were 0.032 mM (given as the monomer concentration) and the biotin-containing samples had a biotin:avidin molar ratio of 3:1. The reference cell was filled with the same buffer that the sample proteins were dissolved in (100 mM Na-phosphate buffer pH 7.4). The thermograms were recorded as a function of temperature, between 25 and 130 °C at a temperature scan rate of 55.6 °C/h. The baselines were subtracted and the transition midpoints (Tₘ) of the samples were calculated using proprietary software provided by the instrument manufacturer.

The results are shown in a baseline-subtracted form (Fig. 3) and numerically (Table 2). As shown by the thermograms, Avd-ci was the most stable avidin mutant since its denaturation Tm value was the highest, both in the absence and presence of biotin. Avd-ccci was only slightly less stable than Avd-ci. The other mutants and wt avidin were equally stable when bound to biotin, whereas Avd-nc and Avd-cci showed the lowest Tm values in the absence of biotin. The heat-induced unfolding of the avidins was an irreversible process and the unfolding of the proteins was of ten followed by a more or less sharp decrease in the heat capacity (due to aggregation, data not shown).

**Table 2. Heat-induced unfolding of the wild-type and mutant avidins with or without biotin was examined using DSC. Values for Tₘ are the averages of the results from two different experiments (± S.E.M.). The value for ΔTₘ indicates the difference in Tm compared to that of wt avidin or wt avidin + biotin.**

| Sample | Tₘ/(°C) | ΔTₘ/(°C) |
|---|---|---|
| wt Avidin | 85.5 ± 0.1 | |
| + biotin | 118.2 ± 0.1 | |
| | | |
| Avd-nc | 76.4 ± 0.4 | -9.1 |
| + biotin | 118.5 ± 0.1 | 0.3 |
| | | |
| Avd-ci | 98.6 ± 0.1 | 13.1 |
| + biotin | 124.7 ± 0.5 | 6.5 |
| | | |
| Avd-cci | 74.4 ± 0.1 | -11.1 |
| + biotin | 117.5 ± 0.1 | -0.7 |
| | | |
| Avd-ccci | 94.7 ± 0.5 | 9.2 |
| + biotin | 122.0 ± 0.1 | 3.8 |

### Example 5.

### Sequence comparison and molecular modelling of AVR proteins

The EMBL database accession numbers for the AVR genes used to deduce the corresponding amino acid sequences are as follows: AVR1, Z21611; AVR2, Z21554; AVR3, Z21612; AVR4, Z22883, AVR6, AJ237658; AVR7, AJ237659. AVR4 represents also the identical AVR5, and we therefore use here the term AVR4/5. The AVR cDNAs were translated with the GCG software package program Map (Genetic Computer Group, Madison, WI, USA). The theoretical pI for the AVRs and avidin was determined by using the GCG-package program Peptidesort. The comparison of the sequences of avidin and AVR1-AVR7 was performed using Malign (Johnson, M. S. and Overington, J. P. (1993) J. Mol. Biol. 233, 716-38; Johnson, M. S., May, A. C., Rodionov, M. A. and Overington, J. P. (1996) Methods Enzymol. 266, 575-98) in the BODIL Modelling Environment (Lehtonen, Rantanen, Still and Johnson, unpublished). Model structures of AVR1-AVR7 were made using the rapid homology modelling program HOMODGE in BODIL on the basis of the x-ray structure of chicken avidin in complex with biotin (PDB code: lavd; (Pugliese, L., Coda, A., Malcovati, M. and Bolognesi, M. (1993) J. Mol. Biol. 231, 698-710). HOMODGE aims to reduce errors in models of closely related proteins by using as many atomic coordinates from the known structure as possible to build the conserved main chain and side chain structures. Where the side-chains differ, HOMODGE uses a rotamer library to select the side chain conformation in order to optimise contacts.

The putative AVRs were compared to avidin to identify differences that might affect their biotin binding, structural or other biochemical properties. The biotin-binding residues are well conserved in all AVR proteins, showing only three amino acid changes (out of the 16 possible) (Figure 4). Three sequential residues involved in biotin binding (38-40, Thr-Ala-Thr in avidin) are substituted by Ala-Asp-Asn in all AVR proteins. With the exception of AVR7, the AVR proteins lack the glycosylation site (Asn-17) present in avidin. Instead, they all exhibit other Asn-Xaa-Ser/Thr sequences, which could be glycosylated during protein maturation along the secretion route. There is one common Asn-Xaa-Ser site in the L5 loop of all the AVR proteins. Additional Asn-Xaa-Ser/Thr sequences are distributed as follows: two in AVR1 (in strand β3 and in L3), one in AVR2 (L3), one in AVR3 (β8), two in AVR4/5 (L3 and β8), and two in AVR6 and AVR7 (β3 and β8). The potential N-glycosylation sites of the AVRs are located on the surface of the tetramers and, therefore, they are expected to be glycosylated.

The theoretical pIs of AVR3 and AVR4/5 are basic, similar to the pI of avidin (avidin 10.4, AVR3 10.2 and AVR4/5 10.0). On the other hand, AVR1, AVR6 and AVR7 are neutral (pI 7.3) and AVR2 is calculated to be acidic with a pI of 4.7. All AVR proteins, except for AVR2, have three cysteine residues (Figure 4). In AVR1, AVR3, AVR6 and AVR7, the third "extra" cysteine replaces Thr-60 of avidin located at the beginning of β5. In AVR4/5, the "extra" cysteine residue is close to the C-terminus of the protein where Arg-124 is found in avidin. This extra cysteine causes agglomeration of the tetramers and thus it would be preferable to substitute it with another amino acid.

The interface regions between different subunits show a variable number of amino acid substitutions between the AVR proteins and avidin. The interface between subunits 1 and 2 (as well as between 3 and 4) is perfectly conserved in all of the AVRs. In contrast, two out of three interface residues between subunits 1 and 3 (2 and 4) show changes in all of the AVR proteins, except for AVR4/5, which shows only one substitution (Ile-117-Tyr). Interestingly, the tightly interacting 1-4 (2-3) subunit interface shows several amino acid substitutions in all of the AVRs. In avidin, a total of 22 residues confer intersubunit contacts within this interface. The AVR proteins have nine substitutions in this region, seven of which are found in all AVRs and two in all AVRs but AVR4/5 (Table 3).

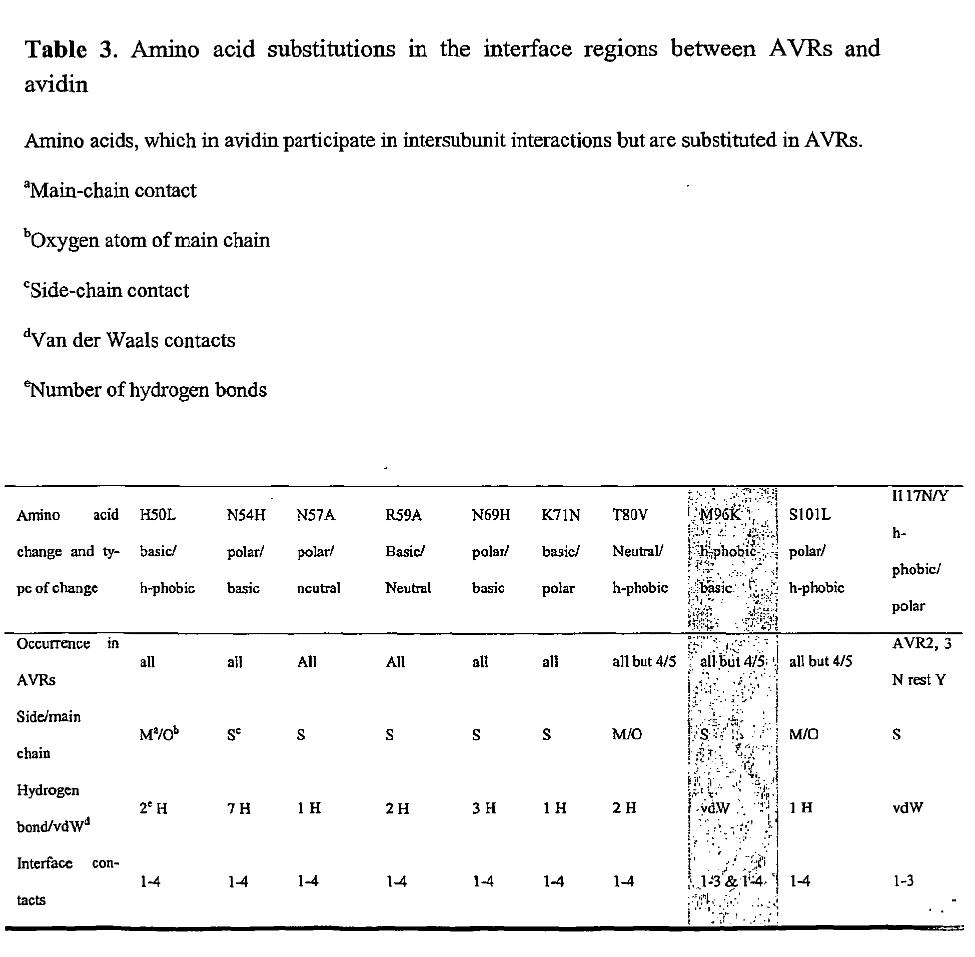

Most of the sequence differences in the subunit interface of avidin and AVR1-AVR7 would not interfere with tetramer formation. The most critical sequence difference is located at position 96 where methionine in avidin and AVR4/5 is replaced by lysine in AVR1-AVR3 and AVR6-AVR7. In avidin and AVR4/5, Met-96 interacts with Met-96 from a second monomer of the tetramer (Figure 5a). When Met-96 is replaced by the positively-charged lysine as seen in AVR1-AVR3 and AVR6-AVR7, it would be logical to expect that charge repulsion interferes with the formation of the tetramer. However, this does not seem to occur but, instead, Lys-96 from monomer 1 can form hydrogen bonds with the side-chain OH-group of Thr-113 from monomer 4, and with the main-chain oxygen of Val-115 from monomer 3 (Figure 5b).

As compared to avidin, the biotin-binding pocket is highly conserved in AVR1-AVR7, where only minor changes in the sequences and therefore in the shape of the binding pocket are observed. These changes are limited to the area surrounding the most flexible part of the biotin molecule and, thus, biotin can easily compensate for these changes. The only exception occurs at position 111. In avidin, AVR1 and AVR3-AVR7 lysine is found at this position, whereas in AVR2 isoleucine is present. In the avidin crystal structure, the hydrophobic part of the lysine side-chain interacts with the indole-ring of Trp-110 keeping its position fixed (Figure 5c). The replacement of Lys-111 by the bulky isoleucine side chain would force the side chain of Trp-110 either to bend towards the binding pocket (effectively blocking biotin entry to the site) or away from the binding pocket (allowing the entry and exit of biotin into/from the site) (Figure 5d). Trp-110 is one of the most critical amino acids in the binding pocket because it conforms exactly to the shape of biotin where the ligand is most rigid. The N-linked glycosylation site on L5, although close to the biotin binding site, is solvent-exposed. The asparagine that is glycosylated is not in the vicinity of the ligand, whereas the serine side chain is hydrogen bonded to the carboxylate group of biotin. When biotin is absent (for example, during post-translational glycosylation) the serine side chain should be accessible to the glycosylating enzyme.

All AVRs, with the exception of AVR2, contain a third single cysteine (in addition to the two present in avidin) located on the surface of the proteins. Since AVR2 does not have the extra cysteine, there is no reason to believe that AVR2 could form tetramers in a way that is different to avidin. However, in AVR1, AVR3, AVR6 and AVR7, the extra cysteine is located in L4 at position 60 (avidin numbering), where it is impossible to form an extra disulfide-bond between subunits without severely disturbing tetramer formation.

### Example 6.

### Production and purification of recombinant AVR proteins

The AVR1-4/5 genes (Keinänen, R. A., Laukkanen, M. L. and Kulomaa, M. S. (1988) J. Steroid Biochem. 30, 17-21; Keinänen, R. A., Wallen, M. J., Kristo, P. A., Laukkanen, M. O., Toimela, T. A., Helenius, M. A. and Kulomaa, M. S. (1994) Eur. J. Biochem. 220, 615-21) were cloned into the EcoRI-HindIII sites of pGEM4 and were in vitro transcribed and spliced using Ribomax and RNA Splicing System kits (Promega) according to the manufacturer's instructions. The cDNAs were then produced by RT-PCR and further amplified by PCR using the oligonucleotide primers: AK33 (5'-CTGCTAGATCTATGGTGCACGCAACCTCCCC-3') and AK44 (5'-GTTGCAAGCTTTGCGGGGCCATCCT-3') containing BgIII and HindII restriction sites, respectively. After cutting with BglII and HindIII, the AVR1-4/5 cDNAs were cloned into BamHI and HindIII sites of pFASTBAC. For AVR6 and AVR7, cDNAs were produced by subcloning the corresponding genes (Ahlroth, M. K., Kola, E. H., Ewald, D., Masabanda, J., Sazanov, A., Fries, R. and Kulomaa, M. S. (2000) Anim. Genet. 31, 367-75) into pDsRed1 (Clontech) where the red fluorescent protein-encoding region had been removed, and by subsequent transfection of the constructs into NIH/3T3 cells. Total RNA was extracted from the cells using the SV Total RNA Isolation System (Promega). The AVR6 and AVR7 cDNAs were produced by RT-PCR (RobusT RT-PCR Kit, Finnzymes, Espoo, Finland) using the oligonucleotide primers AK33 and AK44 to produce BglII and HindIII restriction sites to the 5'- and 3'-ends of the cDNAs, respectively. The synthesised cDNAs were cloned into BamHI/HindIII digested pFASTBAC1, the cloning vector for the Bac-To-Bac Baculovirus Expression System (Gibco BRL, Life Technologies, Gaithersburg, MD, USA). The nucleotide sequences of the cDNAs were confirmed by sequencing. The virus vectors for producing the AVR proteins were constructed and amplified according to the Bac-To-Bac system instructions. Recombinant AVR proteins were produced in Sf9 insect cells as previously reported (Airenne, K. J., Oker-Blom, C., Marjomäki, V. S., Bayer, E. A., Wilchek, M. and Kulomaa, M. S. (1997) Protein Expr. Purif. 9, 100-10822). Proteins were purified from the cells using affinity chromatography on a 2-iminobiotin (AVR1, AVR3, AVR4/5-7) and/or biotin agarose column (AVR1 and AVR2) as previously described (Laitinen, O. H., Airenne, K. J., Marttila, A. T., Kulik, T., Porkka, E., Bayer, E. A., Wilchek, M. and Kulomaa, M. S. (1999) FEBS Lett. 461, 52-8). AVR2 was eluted from biotin agarose with 1 M acetic acid and the elution fractions were immediately neutralised with NaOH. The elution of AVR1 was achieved using 1 M HCl followed by neutralisation with Tris (1 g/ml).

All AVRs showed different patterns in SDS-PAGE as compared to avidin (Figure 8). One-step 2-iminobiotin (AVR1, AVRs 3-7) and/or biotin (AVRs 1 and 2) agarose affinity chromatography yielded highly homogenous proteins showing no contaminants as judged by SDS-PAGE analysis.

### Example 7.

### Biotin-binding analyses of AVRs

The biotin-binding characteristics of the AVR proteins were studied as previously described using the IASyS optical biosensor (Marttila, A. T., Airenne, K. J., Laitinen, O. H., Kulik, T., Bayer, E. A., Wilchek, M. and Kulomaa, M. S. (1998) FEBS Lett. 441, 313-317; Laitinen, O. H., Airenne, K. J., Marttila, A. T., Kulik, T., Porkka, E., Bayer, E. A., Wilchek, M. and Kulomaa, M. S. (1999) FEBS Lett. 461, 52-8). Affinities were measured for 2-iminobiotin or biotin at 20.0 °C using a stirring power of 100 %. Briefly, the protein under study in biotin-free buffer was allowed to bind to the biotin-coated surface of the IASyS cuvette. As a negative control, the protein under study was saturated with excess biotin prior to (and was also maintained under biotin excess upon) addition to the cuvette. Under these conditions, residual binding to the biotin surface of the cuvette could be regarded as unspecific, and was used as a measure of baseline variation. Binding equilibrium in the experimental cuvette was considered to be attained after no binding was detected as compared to negative control.

The reversibility of biotin binding was studied as follows: Avidin and the AVRs were allowed to bind to a biotin-coated cuvette. After measuring the initial binding (A) as described above, biotin-saturated (0.17 mg/ml) buffer was injected into the cuvette and the amount of proteins remaining bound was measured until no dissociation was seen, again as compared to the negative control cuvette (B). The measurement time was at least 20 minutes. The percent reversibility was calculated as follows: reversibility (%) = 100 * (A-B)/A. A-B is the amount of liberated proteins after biotin adding.

In the reversibility assay, AVRs 3-7 showed totally irreversible biotin binding, similarly to avidin. In contrast, AVR1 exhibited 18 % and AVR2 93 % reversibility following addition of free biotin (Table 4). The actual dissociation constants (Kd) were calculated for the AVRs according to the kass and kdiss rates measured in a separate assay in different protein concentrations (varying between 10 µM and 100 pM) (Table 4 and Figure 6). The binding seemed to follow two-order or even more complex kinetics. However, the first 1000 seconds of the binding fitted considerably well to the bi-phasic curve. All measured dissociation rate constants (kdiss) followed the first-order kinetics. Because of the extremely high binding, affinities of the AVRs for biotin could not be determined except for AVR1, AVR2 and AVR7. Binding to 2-iminobiotin was determined only for AVR4/5, and it was similar to that of avidin. The lack of binding for the other AVRs was surprising, since most of them were purified using 2-iminobiotin agarose. This may be due to the relatively short linker between 2-iminobiotin and the activated group of the IASyS cuvette that did not allow the 2-iminobiotin to penetrate deep enough into the biotin-binding pocket of the other AVRs.

**Table 4. Optical biosensor data for biotin and 2-iminobiotin bindings for avidin and AVRs**

| | | | | | | | |
|---|---|---|---|---|---|---|---|
| Biotin-binding properties of AVRs compared to avidin (AVD), determined using the IASyS biosensor. The values for avidin are from Laitinen et al. (Laitinen, O.H. et al., Biochem. J 363, 609-17 (2002)) and Marttila et al. (Marttila, A.T. et al., FEBS Lett 467, 31-6 (2000)). Reversibility of binding was determined by using a biotin cuvette. | | | | | | | |

| | AVD | AVR1 | AVR2 | AVR3 | AVR4/5 | AVR6 | AVR7 |
|---|---|---|---|---|---|---|---|
| K_{d}(M)^{c} | (1.7 ± 1.3) x 10^{-8a} | (1.0±0.8) x 10^{-7b} | (1.7 ± 1.5) x 10^{-6b} | <<10^{-8b} | (5.7 ± 2.5) x 10^{-7a} | <<10^{-8b} | (4.5 ± 6.2) 10^{-9b} |
| K_{d} (M)^{d} | (2 ± 0.9) x 10^{-8a} | (4.4 ± 1.9) x 10^{-8b} | (5.2 ± 1.7) x 10^{-8b} | ND | (9.1 ± 3.6) x 10^{-8a} | ND | ND |
| kₐₛₛ (M⁻¹s⁻¹) | (1.6 ± 0.7) x 10⁴ | (5.5 ± 1.5) x 10⁴ | (1.8 ± 0.1) x 10⁴ | ND | (1.8 ± 0.2) x 10⁵ | ND | (1.8 ± 0.2) x 10⁵ |
| k_{diss} (s⁻¹) | (3.1 ± 1.4) ± 10⁻⁴ | (2.4 ± 0.8) x 10⁻³ | (4.6 ± 1.1) x 10⁻³ | ND | (1.7 ± 0.7) x 10⁻³ | ND | ND |
| Reversibility (%) | 1 ± 1 | 18 ± 10 | 94 ± 3 | 3 ± 2 | 2 ± 2 | 5 ± 3 | 3 ± 3 |

| | | | | | | | |
|---|---|---|---|---|---|---|---|
| aMeasured in a 2-iminobiotin cuvette. bMeasured in a biotin cuvette. cThe dissociation constants were calculated from the equilibrium response data. dThe dissociation constants were calculated directly from the binding curves. ND Not determined. | | | | | | | |

### Example 8.

### Structural analyses of AVRs

The heat stability of the AVRs was studied by using an SDS-PAGE-based method (Bayer, E. A., Ehrlich-Rogozinski, S. and Wilchek, M. (1996) Electrophoresis **17**, 1319-1324). In this method, each protein sample was divided into two fractions one of which was kept biotin-free and the other was supplemented with an excess of biotin. The samples were then mixed with denaturing SDS-PAGE sample buffer (containing β-mercaptoethanol) and incubated at a given temperature for 20 minutes. After heat treatment, the samples were subjected to SDS-PAGE and visualised by staining with Coomassie brilliant blue. The relative proportions of tetrameric and monomeric forms of the AVR proteins (with avidin as the control) were detected. A similar assay, with β-mercaptoethanol omitted from the sample buffer, was performed to examine the presence of intersubunit disulfide-bridges. Sensitivity to proteinase K was studied both in the absence and in the presence of biotin as described (Laitinen, O. H., Airenne, K. J., Marttila, A. T., Kulik, T., Porkka, E., Bayer, E. A., Wilchek, M. and Kulomaa, M. S. (1999) FEBS Lett. 461, 52-8). The isoelectric point of each AVR was determined by isoelectric focusing as previously reported (Marttila, A. T., Airenne, K. J., Laitinen, O. H., Kulik, T., Bayer, E.., Wilchek, M. and Kulomaa, M. S. (1998) FEBS Lett. 441, 313-317). The glycosylation patterns of the AVR proteins were studied by treating the proteins with Endo Hf and PNGase F glycosidase according to the manufacturer's instructions (New England Biolabs, MA, USA).

All AVRs exhibited heat stability comparable to avidin in a reducing SDS-PAGE assay (not shown). In the absence of biotin, AVR tetramers began to dissociate into monomers at around 60 °C. In the presence of biotin, a portion of AVRs remained tetrameric even upon boiling. Non-reducing SDS-PAGE showed that AVR1 and AVRs 3-7 have a tendency to form dimers. Under reducing conditions, only tetramers and monomers could be discerned. This result suggests that the dimers are crosslinked via disulfide bridges (stable enough to hold the dimers together under non-reducing conditions) and are slowly degraded into monomers by heat (Figure 7 and Table 5). The shift from a fully tetrameric state into dimeric and monomeric state was detected upon raising the temperature gradually. AVR2 is an exception since it disintegrated directly into monomers, similarly to native avidin.

The AVRs also showed remarkable resistance against proteolysis. In the presence of biotin, all AVRs remained intact after 16 hours of proteinase K treatment. Proteolysis occurred, albeit slowly, in the absence of biotin. Taken together, AVRs showed stability similar to or even greater than avidin (not shown).

The multiple bands observed for the AVRs in SDS-PAGE were found to be differently glycosylated forms. Treatment with Endo Hf glycosidase and PNGase F eliminated the higher-molecular weight bands. The number of putative glycosylation sites seems to correlate well with the observed glycosylation patterns. Preliminary deglycosylation results using non-denaturing conditions suggest that N-glycan is attached to the glycosylation site in L5 in AVRs 1,2,4/5. Isoelectric focusing showed that the pIs of the AVRs were approximately the same as expected from theoretical calculations; ≈ 7 for AVRs 1, 6, and 7; ≈ 5 for AVR2; and ≈ 10 for AVRs 3, and 4/5.

**Table 5. Comparative thermostability of avidin and AVRs in non-reducing conditions**

| | | | | | | | |
|---|---|---|---|---|---|---|---|
| The relative proportions (as percentage) of different oligomeric forms of AVRs and avidin (AVD) in SDS-PAGE under non-reducing conditions, boiled 20 minutes before loading onto the gel. Note the presence of dimeric forms in the case of AVRs 1, 3-7. | | | | | | | |

| Protein | AVD | AVR1 | AVR2 | AVR3 | AVR4/5 | AVR6 | AVR7 |
|---|---|---|---|---|---|---|---|
| Tetrameric | - | 20 | - | - | - | - | - |
| Dimeric | - | 40 | - | 50 | 50 | 50 | 20 |
| Monomeric | 100 | 40 | 100 | 50 | 50 | 50 | 80 |

### Example 9.

### Mutagenesis of AVR4/5 and the purification of expressed recombinant proteins

Site-directed mutagenesis of cDNA encoding AVR4/5 protein (Laitinen, O. H., Hytönen, V. P., Ahlroth, M. K., Pentikäinen, O. T., Gallagher, C., Nordlund, H. R., Ovod, V., Marttila, A. T., Porkka, E., Heino, S., Johnson, M. S., Airenne, K. J. & Kulomaa, M. S. (2002) Biochem. J. 363, 609-617) was performed by using QuikChange (Stratagene, La Jolla, CA, USA) mutagenesis method.

Based upon the sequence alignment of AVR4/5 and avidin (Figure 4), Asp-43 of AVR4/5 was mutated to glutamate in order to avoid possible glycosylation at that position. Cys-124 in AVR4/5 was converted to serine to eliminate the formation of extraneous disulfide bridges in AVR4/5. Serine was chosen due to sequence differences that AVR4/5 and avidin show at their C-termini. Serine is a polar residue having a similar size as cysteine. The resulting mutants were designated as AVR4/5(N43E) and AVR4/5(C124S) and they were produced as described in (Laitinen, O. H., Hytönen, V. P., Ahlroth, M. K., Pentikäinen, O. T., Gallagher, C., Nordlund, H. R., Ovod, V., Marttila, A. T., Porkka, E., Heino, S., Johnson, M. S., Airenne, K. J. & Kulomaa, M. S. (2002) Biochem. J. 363, 609-617). Tyr-117 in AVR4/5(C124S) was converted to Cys in order to raise a double mutant AVR4/5(Y117C,C124S) and enable the formation of disulphide bridges between subunits in the same tetramer (Nordlund, H. R., Laitinen, O. H., Uotila, S. T., Nyholm, T., Hytönen, V. P., Slotte, J. P. & Kulomaa, M. S. (2003) J. Biol. Chem. 278, 2479-2483).

Recombinant baculoviruses producing AVR4/5 and its mutated forms were generated according to instructions of Bac-To-Bac^{™} -system (Gibco BRL, Life Technologies, Gaithersburg, MD, USA). Wild type and mutant AVR4/5 proteins were produced in baculovirus infected Sf9 insect cells in biotin-free medium as reported earlier (Laitinen, O. H., Hytönen, V. P., Ahlroth, M. K., Pentikäinen, O. T., Gallagher, C., Nordlund, H. R., Ovod, V., Marttila, A. T., Porkka, E., Heino, S., Johnson, M. S., Airenne, K. J. & Kulomaa, M. S. (2002) Biochem. J. 363, 609-617). The produced proteins were purified with affinity chromatography using 2-iminobiotin agarose as previously described (Laitinen, O. H., Marttila, A. T., Airenne, K. J., Kulik, T., Livnah, O., Bayer, E. A., Wilchek, M. & Kulomaa, M. S. (2001) J. Biol. Chem. 276, 8219-8224).

Briefly, the insect cell infections were carried out with cells that were transferred to a biotin-free medium. Infection was allowed to proceed for 72 hours, and after that the cells were collected by centrifugation (1500 g, 5 min). The cell pellet was resuspended into a lysis buffer (50 mM Tris-HCl, 2 mM EDTA, 150 mM NaCl, 1 % Triton X-100, pH 8) and incubated on ice for 30 minutes. After that the cell extract was sonicated and centrifuged (15000 g 20 min) and the pH of the supernatant was adjusted to 11 with 5 M NaOH and NaCl was added to a final concentration of 1 M. Then the cell extract was applied to a 2-iminobiotin agarose column washed previously with binding buffer (50 mM Na-carbonate, 1 M NaCl). Bound proteins were eluted from the column with 50 mM Na-acetate, pH 4.

The concentrations of the avidin solutions were calculated from the absorbance at 280 nm using an extinction coefficient of 24280 M-1cm-1 for avidin, 26960 M-1cm-1 for AVR4/5 and AVR4/5(N43E), 26840 M-1cm-1 for AVR4(C124S) and 25680 M-1cm-1 for AVR4/5(Y117C, C124S) (Green, N. M. (1975) Adv. Protein Chem. 29, 85-133).

### Example 10.

### Glycosylation analysis of AVR 4/5 and its mutants

The glycosylation patterns of the proteins were studied by treating the proteins with Endo Hf glycosidase according to the manufacturer's instructions (New England Biolabs, MA, USA) as described in (Laitinen, O. H., Hytönen, V. P., Ahlroth, M. K., Pentikäinen, O. T., Gallagher, C., Nordlund, H. R., Ovod, V., Marttila, A. T., Porkka, E., Heino, S., Johnson, M. S., Airenne, K. J. & Kulomaa, M. S. (2002) Biochem. J. 363, 609-617). Samples were then analyzed with 15 % SDS-PAGE and stained with Coomassie Brilliant Blue. Non-treated protein samples were used as a control.

Treatment with Endo Hf glycosidase caused the disappearance of higher molecular weight bands in the protein samples (Figure 11A). In the case of AVR4/5(N43E), there was no band corresponding to the highest molecular weights form present in untreated samples of AVR4/5.

### Example 11.

### Gel filtration analysis of AVR 4/5 and its mutants

The oligomeric state of the AVR4/5 and its mutant forms were assayed with FPLC gel filtration using Shimadzu HPLC liquid chromatography equipped with a Superdex 200 HR 10/30 column (Amersham Pharmacia Biotech, Uppsala, Sweden) and fluorescence detector (excitation 280 nm, detection 350 nm). The column was calibrated using the gel filtration mixture (thyroglobulin, IgG, ovalbumin, myoglobin, vitamin B-12; Bio-Rad Laboratories, Hercules, CA, U.S.A) and bovine serum albumin (Roche Diagnostics, Mannheim, Germany) as molecular weight standards. Sodium phosphate buffer (50 mM, pH 7.2) or sodium carbonate buffer (50 mM, pH 11) with 650 mM NaCl was used as the liquid phase. Protein samples varying between 5 and 25 µg in a volume of 20 µl were used in the analysis. The reduction of intertetrameric disulphide bridges were performed by adding 2-mercaptoethanol to final concentration of 10 % (v/v) after which the protein sample was incubated 1 h at 37 °C.

AVR4/5 and its mutated form N43E appeared to be heterogeneous in gel filtration, since higher molecular weight forms were seen (Figure 10A, Table 6). The oxidization of AVR4/5 caused extensive oligomerisation of the protein (Figure 10C). When sample of AVR4/5 was treated with 2-mercaptoethanol the high molecular weight forms disappeared and the protein existed mainly in the tetrameric form (Figure 10D). AVR4/S(C124S) appeared as a tetramer (Figure 10B) identically to avidin. AVR4/5(Y117C,C124S) appeared as a tetramer, which indicates the absence of intertetrameric disulphide bridges (present in native AVR4/5) (Figure 10E).

**Table 6. Structural properties of avidins.**

| | | | | | | | |
|---|---|---|---|---|---|---|---|
| FPLC gel filtration elution times and calculated molecular weights of the proteins. Heat-induced unfolding of proteins determined by DSC (average ± S.D). A review of the previously published results is also included. | | | | | | | |

| **Protein** | **Gel filtration** | | **DSC** | | | | |
|---|---|---|---|---|---|---|---|
| | **Elution time(s) (min)** | **Molecular weight (kDa)** | **Tₘ(-biotin) (°C)** | **Tₘ (+biotin) (°C)** | **ΔTm^{a} (°C)** | **ΔH^{b} (kJ/mol)** | **ΔCₚ (kJ/K mol)** |
| **Chicken avidin** | 28.5 | 61.4 | 83.5 ± 0.1 | 117.0 ± 0.7 | 33.5 | 329 ± 5 | 15.3 |
| **AVR4/5^{c}** | 28.9 | 55.1 | 107.4 ± 0.3 | 124.7 ± 0.3 | 17.3 | 811 ± 140 | n.m.^{d} |
| | 25.6 | 121.6 | | | | | |
| | 23.5 | 200.5 | | | | | |
| **AVR4/5(N43E)^{c}** | 29.5 | 48.3 | 104.9 ± 0.3 | n.d.^{e} | n.d.^{e} | n.d.^{e} | n.m. |
| | 26.0 | 110.3 | | | | | |
| | 23.9 | 186.2 | | | | | |
| **AVR4/5(C124S)** | 29.0 | 54.8 | 106.4 ± 0.8 | 125.4 ± 0.8 | 19.5 | 575 ± 33 | 9.1 |
| | | | | | | | |
| **AVR4/5 + 2-mercaptoethanol** | 28.9 | 55.1 | 106.5 ± 0.3 | 125.2 ± 0.1 | 18.7 | 708 ± 26 | n.m. |
| **AVR4/5(Y117C, C124S)^{f}** | 29.0 | 54.8 | 109.9 ± 0.2 | 123.3 ± 0.1 | 13.4 | | |
| **AVD-ci^{g}** | | | 98.6 ± 0.1 | 124.7 ± 0.5 | 26.1 | | |
| **AVD-cci^{g}** | | | 74.4 ± 0.1 | 117.5 ± 0.1 | 43.1 | | |
| **AVD-ccci^{g}** | | | 94.7 ± 0.5 | 122.0 ± 0.1 | 27.3 | | |
| **AVD-nc^{g}** | | | 76.4 ± 0.4 | 118.5 ± 0.1 | 42.1 | | |
| **Streptavidin^{h}** | | | 75.5 | 112.2 | 36.7 | 629.0 | |

| | | | | | | | |
|---|---|---|---|---|---|---|---|
| ^{a}ΔTₘ is the change in Tₘ upon addition of a three-fold molar excess of biotin. ^{b}ΔH value obtained from sample without biotin. The value could not be determined accurately from samples saturated with biotin because the Tₘ values were too close to the temperature limit of the DSC equipment. ^{c}Three protein forms with lowest molecular weights are indicated. ^{d}Not measured. ^{e}Not determined due to low signal. ^{f}Unpublished results (V. P. Hytönen 2003) gNordlund, H. R., Laitinen, O. H., Uotila, S. T., Nyholm, T., Hytönen, V. P., Slotte, J. P. & Kulomaa, M. S. (2003) J. Biol. Chem. 278, 2479-2483. hGonzalez, M., Argarana, C. E. & Fidelio, G. D. (1999) Biomol. Eng. 16, 67-72. | | | | | | | |

### Example 12.

### Non-reducing SDS-PAGE analysis of AVR 4/5 and its mutants

Protein samples in the absence or presence of biotin (0.02 mg/ml) were diluted to a final concentration about 0.2 mg/ml in 50 mM Na-carbonate buffer pH 8.7, and proteins were acetylated by adding NHS-acetic acid conjugate to a final concentration of 0.2 µg/ml. The mixture was incubated at room temperature (23 °C) for 10 min. Equal amount of SDS-PAGE sample buffer without 2-mercaptoethanol was added to the sample and it was boiled for 20 min. After analysis by 15 % SDS-PAGE, the gel was stained with Coomassie brilliant blue.

In the SDS-PAGE analysis AVR4/5(Y117C, C124S) (Figure 11C) appeared both in dimeric and monomeric form in the non-reducing environment (dimeric form indicates the formation of disulphide bridges between subunits). (The presence of monomeric forms could indicate some inefficiency in disulphide bridge formation).

### Example 13.

### Optical biosensor studies of AVR 4/5 and its mutants

The biotin-binding characteristics of the different avidins were studied using an IAsys optical biosensor at 20 °C. The reversibility of binding and binding affinities to the 2-iminobiotin surface in 50 mM borate buffer (pH 9.5, 1 M NaCl) were measured as previously reported (Laitinen, O. H., Hytönen, V. P., Ahlroth, M. K., Pentikäinen, O. T., Gallagher, C., Nordlund, H. R., Ovod, V., Marttila, A. T., Porkka, E., Heino, S., Johnson, M. S., Airenne, K. J. & Kulomaa, M. S. (2002) Biochem. J. 363, 609-617).

Briefly, The 2-iminobiotin ligand was immobilized onto the carboxymethyldextran surface of a cuvette using N-hydroxysuccinimide activation. Binding of various concentrations of avidin or avidin mutants onto 2-iminobiotinylated surface were measured in a 50 mM borate buffer (pH 9.5) containing 1 M NaCl at 20 °C. The iminobiotin cuvettes were regenerated with 20 mM HCl. The kinetic rate constants for association (kₐₛₛ) and dissociation (k_{diss}) were calculated using the Fast Fit program package (Affinity Sensors). The obtained rate constants were used to calculate K_{d}(rel) according to K_{d} = k_{diss}/kₐₛₛ. Equilibrium data obtained from measurements with different protein concentrations were used to calculate the dissociation constant K_{d}(eq).

All proteins exhibited in the IAsys interaction analysis strong binding to the biotin surface, similar to that of chicken avidin (Table 7). All AVR4/5 variants showed similar affinities towards the 2-iminobiotin surface (Table 7). These affinities were, however, an order of magnitude lower than that exhibited by avidin for 2-iminobiotin. In all cases, the slower association rate was observed as the reason for the lowered 2-iminobiotin affinities.

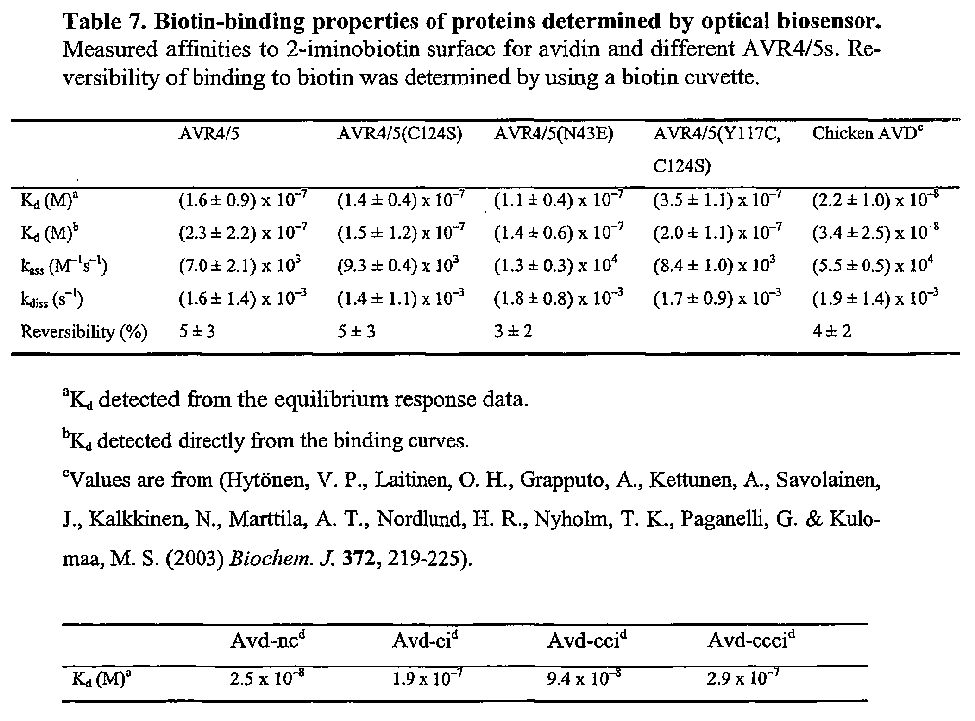

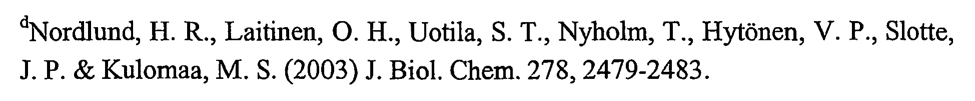

### Example 14.

### Differential scanning calorimetry

The thermodynamics of the heat-induced unfolding of the proteins were studied using DSC.The transition midpoint of heat-induced denaturation (Tm) of the avidin and different AVR4/5 forms was studied using a Nano II differential scanning calorimeter (Calorimetric Sciences Corporation, Provo, UT, USA). The reference cell was filled with buffer whereas the sample cell was filled with proteins dissolved in 50 mM sodium phosphate buffer, pH 7.0 containing 100 mM NaCl. Avidin and AVR4/5(C124S) were also analyzed in 50 mM Na-acetate pH 4.0 containing 100 mM NaCl. Furthermore, AVR4/5 was analyzed in the presence of 2-mercaptoethanol (0.4 % v/v). The samples (1.6-9.6 µM) were analyzed both in the absence and presence of a three fold molar excess of biotin/subunit. All samples were degassed before being loaded into the DSC. The thermograms were recorded as a function of temperature; between 25 and 130 °C at a rate of 55.6 °C/h (Gonzalez, M., Argarana, C. E. & Fidelio, G. D. (1999) Biomol. Eng. 16, 67-72; Nordlund, H. R., Laitinen, O. H., Uotila, S. T., Nyholm, T., Hytönen, V. P., Slotte, J. P. & Kulomaa, M. S. (2003) J. Biol. Chem. 278, 2479-2483). Data was analyzed using Microcal Origin 6.0 and Cpcalc 2.1 (Applied Thermodynamics, Hunt Valley, Maryland). The change in heat capacity upon unfolding (ΔCp) was measured for avidin and AVR4/5(C124S) using DSC data from measurements in pH 4.0 and 7.0. ΔCp was then obtained from the slope of ΔH vs. Tm (Liu, Y. & Sturtevant, J. M. (1996) Biochemistry 35, 3059-3062).

Biotin was obtained Sigma Chemicals and was used as delivered. A 0.395 mM solution of biotin was prepared in the measurement buffer. The concentration of biotin was determined gravimetrically with a microbalance.

In comparison to wild type avidin, all AVR4/5 proteins unfolded at a markedly higher temperature (Table 6). The enthalpy change ΔH upon unfolding was larger for AVR4/5 and AVR4/5(C124S) as compared to avidin. Due to addition of biotin, the Tm was shifted to a higher temperature for all proteins. None of the AVR4/5 forms were, however, stabilized to the same degree as wild type avidin (ΔTm, Table 6). Treatment with 2-mercaptoethanol caused no significant decrease in the Tm of AVR4/5.

The Tm values of avidin and AVR4/5(C124S) at pH 4 were 76.6 ± 0.2 °C and 103.2 ± 0.5 °C, respectively. The change in heat capacity upon unfolding (ΔCp) was rather similar for AVR4/5(C124S) and avidin (Table 6). The ΔCp for avidin was similar to the value reported by Donovan and Ross (Donovan, J. W. & Ross, K. D. (1973) Biochemistry 12, 512-517).

### Example 15.

### Isothermal titration calorimetry

Binding enthalpies (ΔH) where measured with an isothermal titration calorimeter (ITC-4200, Calorimetric Sciences Corporation, Provo, UT, USA). The ITC was calibrated chemically by titrating aliquots of 1.00 mM HCl into 265 mM Tris dissolved in water. The measurements where performed at 25 and 37 °C with 1.0 ml protein solution (20-25 µM) kept under constant stirring. Biotin (0.395 mM) was titrated into the sample cell in 10 µL aliquots. The heat of dilution, which was small compared to the heat of the binding reaction, was corrected for in the analysis of the results. Analysis of the data was performed using Titration Bindworks 3.0.69 (Applied Thermodynamics, Hunt Valley, MD, USA). ΔCpL was calculated from the slope of ΔH vs. temperature (T).

The interaction between AVR4/5(C124S) and biotin was compared to that between avidin and biotin using ITC. Because AVR4/5(C124S) was found to bind biotin as AVR4/5 and to oligomerize like avidin, it was selected for ITC measurements. As these proteins have a very high affinity for biotin, no binding constant could be calculated from the ITC experiments alone. However, since all ligand is bound in the titration, the binding enthalpy could be calculated from the heat released upon the injection of biotin. The ITC experiments, performed at 25 and 37 °C, showed that both the ΔH and ΔCp were similar for biotin binding to AVR4/5(C124S) and avidin (Table 6). The binding enthalpy for biotin binding to avidin was similar to a previously reported value, and the ΔCpL was close to the reported value too (Swamy, M. J. (1995) Biochem. Mol. Biol. Int. 36, 219-225).

The estimated binding constant K_{b} calculated from a combination of ITC and DSC data showed that the binding of biotin to AVR4/5(C124S) (K_{b} ≈2.8 × 1013 M-1) was almost two orders of magnitude lower than for avidin (K_{b} ≈9.3 × 1015 M-1). The difference in how biotin binds to AVR4/5(C124S) and avidin may be interpreted from the thermodynamic data in Table 6. Since ΔH is rather similar for biotin binding to both AVR4/5(C124S) and avidin, the difference in ΔG is expected to be mainly due to an entropic contribution.

### Example 16.

### Microplate assay

Biotin-binding activity of the proteins after heat treatment was studied with a microtiter plate assay (Nordlund, H. R., Laitinen, O. H., Uotila, S. T., Nyholm, T., Hytönen, V. P., Slotte, J. P. & Kulomaa, M. S. (2003) J. Biol. Chem. 278, 2479-2483). Protein samples, 5 µg/ml in PBS (10 mM Na2HP04, 1.8 mM KH2P04, 140 mM NaCl, 2.7 mM KCl, pH 7.2), were heated for various time periods at 99.9 °C (T3 thermocycler, Whatman Biometra, Göttingen, Germany; lid temperature 102 °C, slope 0.2 °C/sec) and then chilled on ice. The samples were transferred to a 96-well Maxisorp-plate (Nalge Nunc International) and incubated at 37 °C for 2 h. The wells were washed three times with PBS-Tween (0.05 % Tween-20 v/v). The wells were then blocked with PBS-BSA (1 % w/v) at 37 °C for 30 minutes and washed again with PBS-Tween. Biotinylated alkaline phosphatase (Sigma) in PBS-BSA was added and incubated at 37 °C for one hour. Once again, the wells were washed with PBS-Tween and the para-nitrophenyl phosphate in DEA buffer (Sigma, 1 mg/ml) was applied to the wells. The absorbencies at 405 nm were measured after incubation for 45 minutes.

The microplate assay showed that AVR4/5 and its mutants resist boiling and remain in an active form significantly longer than chicken avidin or even a stabilized avidin AVD-ci with additional intermonomeric disulphide bonds (Nordlund, H. R., Laitinen, O. H., Uotila, S. T., Nyholm, T., Hytönen, V. P., Slotte, J. P. & Kulomaa, M. S. (2003) J. Biol. Chem. 278, 2479-2483). Of the studied proteins AVR4/5(C124S) showed the greatest stability, since there was about 30 % of the initial binding activity present after boiling for 32 minutes (Figure 12).

### Example 17.

### Structural modeling and sequence analysis of AVR4/5

The three-dimensional structure of avidin in complex with biotin (PDB access code: 1avd;) (Pugliese, L., Coda, A., Malcovati, M. & Bolognesi, M. (1993) J. Mol. Biol. 231, 698-710) was obtained from the Protein Data Bank (PDB). The sequence alignment of avidin and AVR4/5 was made using MALIGN (Johnson, M. S., Overington, J. P. & Blundell, T. L. (1993) J. Mol. Biol. 231, 735-752) in the BODIL Modeling Environment1 using a structure-based sequence comparison matrix (Johnson, M. S., May, A. C., Rodionov, M. A. & Overington, J. P. (1996) Methods Enzymol. 266, 575-598) with gap-penalty 40.

The program HOMODGE in BODIL was used to construct 3D model structures by keeping the side-chain conformations of all identical residues fixed and by maintaining the corresponding torsion angles of similar residues in the alignment. The intramolecular interactions of the amino acids at sequence positions that are different from those in the template structure were analyzed by using the amino acid side-chain rotamer library (Lovell, S. C., Word, J. M., Richardson, J. S. & Richardson, D. C. (2000) Proteins 40, 389-408) incorporated within BODIL.

Molecular modeling and sequence analysis identified amino acid substitutions between avidin and AVR4/5 that might explain the observed differences in the stability and functional properties of AVR4/5. For example, Gly-21 and Gly-107 are found in loops 2 and 7 in avidin, but they are not present in AVR4/5. On the other hand, loop 3 of AVR4/S contains the Pro-41-Gly-42 sequence that is not present in avidin (Figure 4).

In Figure 13, sequence differences between avidin and AVR4/5 are highlighted for residues where the side chains are involved in interactions at the subunit interfaces. The largest interface occurs between subunits 1 and 4 (2 and 3), involving 31 amino acid side-chains from both subunits. This interface shows only four sequence differences. Tyr-55 and Ile-56 in avidin correspond to a deletion in AVR4/5 at a loop located between β-strands 4 and 5. In avidin, the hydrophobic side-chain of Ile-56 of subunit 1 is surrounded by the polar main-chain oxygen atoms of Trp-70, Lys-71 and Ser-73 from subunit 4 (Figure 13A). In AVR4/5, it is likely that the deletion allows favorable interactions between the positively charged side-chain of Arg-56 and the polar main-chain oxygen atoms of Trp-68, Asn-69 and Ser-71 (Figure 13B). In the avidin structure, a hydrophobic interaction is formed between Ile-117 from subunit 1 and Ile-117 from subunit 3 (or subunits 2 and 4). In avidin, Ile-117 is surrounded by several water molecules, reflecting the neighboring polar environment (Figure 13C). In AVR4/5 (Figure 13D), Tyr-117 can form an additional favorable interaction with Tyr-117 from the other subunit. Tyr-117 in AVR4/5 most likely would displace two water molecules and interact with the remaining water molecules and surrounding amino acid residues.

Asp-39 in AVR4/5 corresponds to alanine in avidin being located at the interface between subunits 1 and 2. The side chain of alanine in the avidin structure is unable to form any intramolecular interactions, but only lines the ligand-binding pocket (Figure 14A). In contrast, Asp-39 in AVR4/5 most likely forms a salt bridge with Arg-112 from the same subunit, which could restrict the size of the entrance to the ligand-binding cavity (Figure 14B). In addition, this aspartate can interact with the positively charged end of the Lys-109 side chain from a neighboring monomer.

### Example 18.

### Microparticle analysis of AVR4/5

Before the analysis, 3.22 µm microparticles with COOH-group/0.852 nm2 (Bangs laboratories, Fishers, IN, USA) were covalently coated with biotin-binding proteins. Microparticles (1*108 pcs) were washed twice with H20 by sequential centrifuging (5000 g; 3 min) and aspiration, and resuspended in 200 µl PB buffer (50 mM NaH2PO4-HCl, pH 3.0). A solution of biotin binding protein (50 µl, 1 mg/ml or 3 mg/ml, 50 mM Na-acetate pH 4.0) was added while vortexing. The suspension was incubated for 1 h (500 rpm; 23 °C). Particles were washed with PB and with MES buffer (50 mM, pH 5.5) and suspended in MES buffer (200 µl). A solution of 1-ethyl-3-(3-dimethylaminopropyl)carbodiimide (Pierce, Rockford, IL, USA) in MES buffer (200 µl, 1 mg/ml) was added while vortexing and the suspension was incubated for one hour (500 rpm; 23 °C). Particles were then washed once with MES-buffer and four times with assay buffer (50 mM Tris-HCl, 10 mM NaN3, 1 mM EDTA, 0.1 % Tween-20, pH 8.0). After the last wash particles were suspended in 100 µl volume and concentration of particles was determined with Multisizer 3 Coulter Counter (Beckman Coulter, Fullerton, CA, USA). Streptavidin coated mircospheres (3.12 µm diameter) were purchased from Bangs laboratories.

Microparticles (5*106 pcs) were suspended in 900 µl of assay buffer and aliquoted in four PCR-tubes (i-iv; 180 µl/aliquot). A solution of BF523-biotin (ArcDia Ltd., Turku, Finland) in N,N-dimethylformamide (Riedel-De-Haen, Seeize, Germany), was diluted with assay buffer to concentration of 200 nM and 20 µl of this solution was added in aliquots (i) and (ii) which were then incubated for 1 h under continuous shaking (22 °C, 1100 rpm Thermomixer Comfort, Eppendorf, Hamburg, Germany). The aliquots (ii) and (iv) were then incubated for 30 minutes at 95 °C (PTC-100 equipped with Hot bonnet^{™} heated lid; M&J Research; Waltham, MA, USA) while the aliquots (i) and (iii) were kept that time in room temperature. The BF523-biotin solution (20 µl, 200 nM) was added to aliquots (iii) and (iv) after thermal treatment. All aliquots were incubated for 2 h (22 °C, 1100 rpm) and transferred in 3 replicates (15 µl) to wells of 384-plate (Greiner Bio-One, Frickenhausen, Germany) and the plate was sealed with adhesive PCR film (Abgene, Surrey, UK). The samples were measured with ArcDia TPX PlateReader (ArcDia Ltd.) (Soini, E., Meltola, N. J., Soini, A. E., Soukka, J., Soini, J. T. & Hanninen, P. E. (2000) Biochem. Soc. Trans. 28, 70-74). In order to improve the precision and accuracy of the results, the individual data points (microparticles) characterized with the focal duration time less that 5 msec were omitted.

The negative control for the assay was prepared by saturating the coated particle with unlabeled biotin (100 pmol/l*106 particles) prior to incubation with BF523-biotin.

The fluorescence emission efficiencies of the BF523-biotin conjugate (200 nM) was determined before and after the incubation (30 min) at 95 °C. The measurements were carried out with the ArcDia TPX PlateReader using six sample replicates (15 µl). The obtained 14.1 % decrease in probe fluorescence was used throughout the analyses to correct the results.

Characteristics of AVR4/5 and AVR4/5(C124S) were studied by coating plastic microparticles with proteins. The capacity of AVR4/5 coated particles was remarkably higher as compared to commercial streptavidin particles and particles coated with wild-type avidin and high-stability (Tm = 98.6 °C) AVD-ci with two intersubunit disulfide bonds (Nordlund, H. R., Laitinen, O. H., Uotila, S. T., Nyholm, T., Hytönen, V. P., Slotte, J. P. & Kulomaa, M. S. (2003) J. Biol. Chem. 278, 2479-2483) (Table 4, i, iii). The specificity of BF523-biotin binding to protein-coated particles was confirmed by saturating the particles with unlabeled biotin prior to analysis.

When particles coated with biotin-binding proteins were heat-treated, AVR4/5 forms showed excellent heat resistance as compared to other proteins (Table 4, iv). AVR4/5 and AVR4/5(C124S) showed 93 % and 75 % of the original signal after treatment, respectively. The streptavidin particles showed 21 % of the original signal after heating. AVD-ci showed higher durability (35 %) as compared to wild-type avidin (9 %). When the biotin-conjugate was added before heat-treatment (ii), all particles showed better durability than treated alone.

## Claims

1. A thermostable avidin mutant having an amino acid sequence of four monomers forming a tetramer, **characterized in that** the avidin is an avidin related protein AVR4/5 of Figure 4, wherein the amino acid residue tyrosine 117 has been changed to cystein, and wherein cysteine 124 has been changed to serine. "

2. An isolated polynucleotide sequence encoding the mutant of claim 1."

## Patentansprüche

1. Thermostabile Avidinmutante mit einer Aminosäuresequenz von vier Monomeren, die ein Tetramer bilden, **dadurch gekennzeichnet, dass** das Avidin ein Avidin-verwandtes Protein AVR4/5 gemäß Figur 4 ist, wobei der Aminosäurerest Tyrosin 117 in Cystein geändert wurde und wobei Cystein 124 in Serin geändert wurde.

2. Isolierte Polynukleotidsequenz, kodierend die Mutante gemäß Anspruch 1.

## Revendications

1. Mutant d'avidine thermostable possédant une séquence d'acides aminés de quatre monomères formant un tétramère, **caractérisé en ce que** l'avidine est une protéine apparentée à l'avidine AVR4/5 suivant la figure 4, dans laquelle le résidu acide aminé tyrosine 117 a été modifié en cystéine et dans laquelle la cysteine 124 a été modifiée en sérine.

2. Séquence polynucléotidique isolée codant pour le mutant selon la revendication 1.
